# FASCICULE DE BREVET EUROPEEN

(11) **EP 1 235 824 B1**
(45) Date de publication et mention de la délivrance du brevet: **19.05.2004**
(21) Numéro de dépôt: 00985303.7
(22) Date de dépôt: 22.11.2000
(51) Int. Cl.: C07D 333/16, C07D 213/30, A61K 31/44, A61K 31/381, A61K 7/48, A61P 17/00

(54) **ANALOGUES DE LA VITAMINE D**
VITAMIN D - ANALOGA
VITAMIN D ANALOGUES

(30) Priorité: 24.11.1999 FR 9914783
(43) Date de publication de la demande: 04.09.2002
(73) Titulaire: Galderma Research & Development, S.N.C., 06560 Valbonne (FR)
(72) Inventeur: BERNARDON, Jean-Michel, F-06650 Le Rouret (FR)
(74) Mandataire: Allab, Myriam
(86) Numéro de dépôt international: PCT/FR2000/003250
(87) Numéro de publication internationale: WO 2001/038320

(56) Documents cités:
- EP-A- 0 776 881
- EP-A- 0 850 909
- WO-A-00/26167

## Description

L'invention concerne, à titre de produits industriels nouveaux et utiles, des composés bi-aromatiques analogues de la vitamine D. Elle concerne également leur procédé de préparation et leur utilisation dans des compositions pharmaceutiques destinées à un usage en médecine humaine ou vétérinaire, ou bien encore dans des compositions cosmétiques.

Les composés selon l'invention ont une activité marquée dans les domaines de la prolifération et de la différenciation cellulaire et trouvent des applications plus particulièrement dans le traitement topique et systémique des affections dermatologiques (ou autres) liées à un désordre de la kératinisation, des affections à composante inflammatoire et/ou immunoallergique et de l'hyperprolifération des tissus d'origine ectodermique (peau, épithélium...), qu'elle soit bénigne ou maligne. Ces composés peuvent en outre être utilisés pour lutter contre le vieillissement de la peau, qu'il soit photoinduit ou chronologique et traiter les troubles de la cicatrisation.

On peut également utiliser les composés selon l'invention dans des compositions cosmétiques pour l'hygiène corporelle et capillaire.

La vitamine D est une vitamine essentielle pour la prévention et le traitement des défauts de minéralisation du cartilage (rachitisme), et de l'os (ostéomalacie), et même de certaines formes d'ostéoporose chez le sujet âgé. Mais, il est maintenant admis que ses fonctions s'étendent bien au delà de la régulation du métabolisme osseux et de l'homéostasie calcique. Parmi celles-ci, peuvent être citées ses actions sur la prolifération et sur la différenciation cellulaire et le contrôle des défenses immunitaires. Leur découverte a ouvert la voie à de nouvelles approches thérapeutiques en dermatologie, cancérologie, ainsi que dans le domaine des maladies auto-immunes et celui des transplantations d'organes ou de tissus.

Un apport thérapeutique efficace s'est longtemps heurté à la toxicité de cette vitamine (hypercalcémie parfois mortelle). Actuellement, des analogues structuraux de la vitamine D sont synthétisés, dont certains ne conservent que les propriétés différenciatrices et n'ont pas d'action sur le métabolisme calcique.
C'est un des buts de la présente invention de proposer de nouveaux composés analogues structuraux de la vitamine D qui montrent une activité sélective sur la prolifération et sur la différenciation cellulaire sans présenter de caractère hypercalcimiant.
Un autre but de la présente invention est de proposer de nouveaux composés analogues de la vitamine D qui soient plus facilement synthétisables et donc plus économiques par rapport à ce qui était connu antérieurement.
Ainsi, la présente invention concerne des composés qui peuvent être représentés par la formule générale (I) suivante : 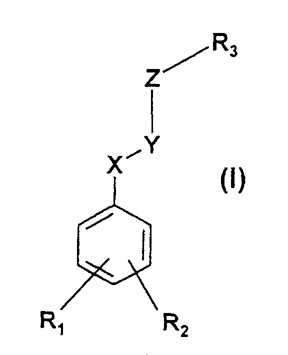 dans laquelle:
- **R**_{**1**} représente un atome d'hydrogène, un radical CH₃ ou un radical -(CH₂)ₛ-OR₄,
- **R**_{**2**} représente un radical -(CH₂)ₜ-OR₅,
   s, t, R₄ et R₅ ayant les significations données ci-après,
- **X-Y** représente une liaison choisie parmi les liaisons de formules (a) à (i) suivantes: 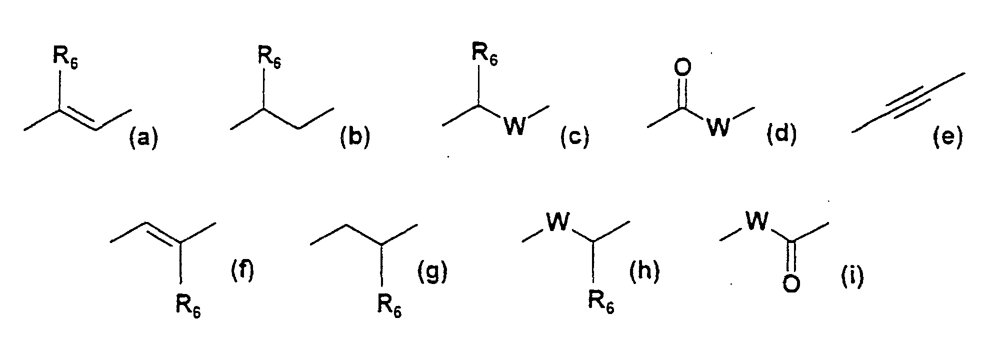 R₆ et W ayant les significations données ci-après,
- **Z** représente un cycle choisi parmi les cycles de formules (**j**) à (**n**) suivantes: 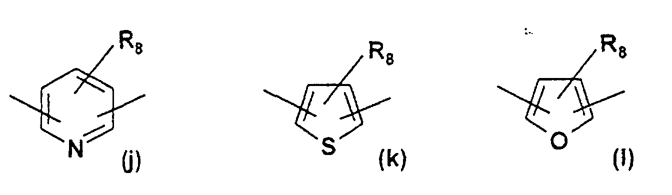 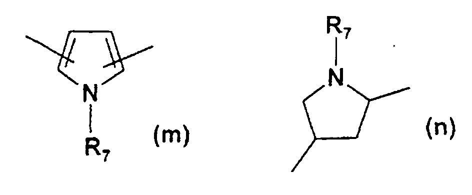 R₇ et R₈ ayant les significations données ci-après,
   étant entendu que lorsque **Z** représente les cycles de formules (**k**), (**l**), ou (**m**), alors **X-Y** ne peut pas représenter une liaison de formule (**c**), (**d**),
   étant entendu que lorsque **Z** représente un cycle de formule (**n**), alors **X-Y** représente de préférence une liaison de formule (**c**), (**d**),
- **R**_{**3**} représente une chaîne alkyle ayant de 4 à 8 atomes de carbone substituée par un ou plusieurs groupements hydroxyle, les groupements hydroxyles pouvant être protégés sous forme d'acétoxy, de méthoxy ou d'éthoxy, de triméthylsilyloxy, de tertiobutyldiméthylsilyloxy, de tétrahydropyranyloxy et éventuellement en outre :
   - substituée par un ou plusieurs groupements alkyles inférieurs ou cycloalkyles et/ou
   - substituée par un ou plusieurs atomes d'halogène et/ou
   - substituée par un ou plusieurs groupements CF₃ et/ou
   - dans laquelle un ou plusieurs atomes de carbone de la chaîne sont remplacés par des atomes d'oxygène, de soufre ou d'azote, les atomes d'azote pouvant être éventuellement substitués par des radicaux alkyles inférieurs et/ou
   - dans laquelle une ou plusieurs liaisons simples de la chaîne sont remplacées par une ou plusieurs liaisons doubles et/ou triples,
   - R3 étant positionné sur le cycle **en para ou méta** de la liaison X-Y,
   - s et t identiques ou différents étant 1 ou 2,
   - R₄ et R₅ identiques ou différents représentent un atome d'hydrogène, un radical acétyle, un radical benzoyle, un radical triméthylsilyle, un radical tertiobutyldiméthylsilyle, ou un radical tétrahydropyranyle,
   - R₆ représente un atome d'hydrogène ou un radical alkyle inférieur,
   - W représente un atome d'oxygène, de soufre ou un radical -NH- pouvant éventuellement être substitué par un radical alkyle inférieur,
   - R₇ représente un atome d'hydrogène ou un radical alkyle inférieur,
   - R₈ représente un atome d'hydrogène, un radical alkyle inférieur ou un atome d'halogène.

L'invention vise également les isomères optiques et géométriques desdits composés de formule (I) ainsi que leurs sels dans le cas où X-Y représentent une liaison de formule (c) et (h) et W représente un radical -NH- éventuellement substitué par un radical alkyle inférieur.

Lorsque les composés selon l'invention se présentent sous forme de sels, il s'agit de sels pharmaceutiquement ou cosmétiquement acceptables obtenus par addition d' un acide minéral ou organique, en particulier l'acide chlorhydrique, sulfurique, acétique, fumarique, hémisuccinique, maléique ou mandélique.

Selon la présente invention on entend par radical alkyle inférieur, un radical linéaire ou ramifié ayant de 1 à 6 atomes de carbone, et de préférence, les radicaux méthyle, éthyle, isopropyle, tertiobutyle et hexyle.
Par radical cycloalkyle, on entend un radical alcane cyclique ou polycyclique contenant de 3 à 10 atomes de carbone. De préférence le radical cycloalkyle est choisi parmi un radical cyclopropyle, cyclopentyle ou cyclohexyle.
Par atome d'halogène, on entend de préférence un atome de fluor, de chlore ou de brome.

Parmi les composés de formule (I) entrant dans le cadre de la présente invention, on peut notamment citer les suivants :
(E)-7-[5-(3,4-Bis-hydroxyméthyl-phenoxyméthyl)-thiophen-2-yl]-3-éthyl-oct-6-en-3-ol
(E)-7-[4-(3,4-Bis-hydroxyméthyl-phenoxyméthyl)-thiophen-2-yl]-3-éthyl-oct-6-en-3-ol
(E)-7-[2-(3,4-Bis-hydroxyméthyl-phenoxyméthyl)-thiophen-4-yl]-3-éthyl-oct-6-en-3-ol
(E)-7-[5-(3,4-Bis-hydroxyméthyl-phenoxyméthyl)-pyridin-3-yl]-3-éthyl-oct-6-en-3-ol
(E)-7-[6-(3,4-Bis-hydroxyméthyl-phenoxyméthyl)-pyridin-2-yl]-3-éthyl-non-6-en-3-ol
(E)-7-[5-(3,4-Bis-hydroxymethyl-phenoxymethyl)-thiophen-2-yl]-3-ethyl-non-6-en-3-ol
(4E,6E)-7-[5-(3,4-Bis-hydroxymethyl-phenoxymethyl)-thiophen-2-yl]-3-ethyl-nona-4,6-dien-3-ol
(3E,5E)-6-[5-(3,4-Bis-hydroxymethyl-phenoxymethyl)-thiophen-2-yl]-1,1,1-trifluoro-2-trifluoromethyl-octa-3,5-dien-2-ol
(4E,6E)-7-[5-(3,4-Bis-hydroxymethyl-phenoxymethyl)-thiophen-2-yl]-1,1,1,2,2-pentafluoro-3-pentafluoroethyl-nona-4,6-dien-3-ol
(E)-7-[5-(3,4-Bis-hydroxymethyl-phenoxymethyl)-thiophen-3-yl]-3-ethyl-4,4-dimethyl-non-6-en-3-ol
(E)-7-{5-[2-(3,4-Bis-hydroxymethyl-phenyl)-ethyl]-thiophen-2-yl}-3-ethyl-non-6-en-3-ol
(4E,6E)-7-{5-[2-(3,4-Bis-hydroxymethyl-phenyl)-ethyl]-thiophen-2-yl}-3-ethyl-nona-4,6-dien-3-ol
(3E,5E)-6-{5-[2-(3,4-Bis-hydroxymethyl-phenyl)-ethyl]-thiophen-2-yl}-1,1,1-trifluoro-2-trifluoromethyl-octa-3,5-dien-2-ol
((4E,6E)-7-{5-[2-(3,4-Bis-hydroxymethyl-phenyl)-ethyl]-thiophen-2-yl}-1,1,1,2,2-pentafluoro-3-pentafluoroethyl-nona-4,6-dien-3-ol
(4E,6E)-7-[5-(3,4-Bis-hydroxymethyl-benzylamino)-thiophen-2-yl]-3-ethyl-nona-4,6-dien-3-ol
(4E,6E)-7-{5-[(3,4-Bis-hydroxymethyl-benzyl)-methyl-amino]-thiophen-2-yl}-3-ethyl-nona-4,6-dien-3-ol
(4E,6E)-7-{5-[(3,4-Bis-hydroxymethyl-benzyl)-propyl-amino]-thiophen-2-yl}-3-ethyl-nona-4,6-dien-3-ol
(E)-7-[4-(3,4-Bis-hydroxymethyl-phenoxymethyl)-thiophen-2-yl]-3-ethyl-non-6-en-3-ol
(4E,6E)-7-[4-(3,4-Bis-hydroxymethyl-phenoxymethyl)-thiophen-2-yl]-3-ethyl-nona-4,6-dien-3-ol
(3E,5E)-6-[4-(3,4-Bis-hydroxymethyl-phenoxymethyl)-thiophen-2-yl]-1,1,1-trifluoro-2-trifluoromethyl-octa-3,5-dien-2-ol
(E)-7-{4-[2-(3,4-Bis-hydroxymethyl-phenyl)-ethyl]-thiophen-2-yl}-3-ethyl-non-6-en-3-ol
(4E,6E)-7-{4-[2-(3,4-Bis-hydroxymethyl-phenyl)-ethyl]-thiophen-2-yl}-3-ethyl-nona-4,6-dien-3-ol
(3E,5E)-6-{4-[2-(3,4-Bis-hydroxymethyl-phenyl)-ethyl]-thiophen-2-yl}-1,1,1-trifluoro-2-trifluoromethyl-octa-3,5-dien-2-ol
(E)-7-[5-(3,4-Bis-hydroxymethyl-phenoxymethyl)-thiophen-3-yl]-3-ethyl-non-6-en-3-ol
(4E,6E)-7-[5-(3,4-Bis-hydroxymethyl-phenoxymethyl)-thiophen-3-yl]-3-ethyl-nona-4,6-dien-3-ol
(3E,5E)-6-[5-(3,4-Bis-hydroxymethyl-phenoxymethyl)-thiophen-3-yl]-1,1,1-trifluoro-2-trifluoromethyl-octa-3,5-dien-2-ol
(E)-7-{5-[2-(3,4-Bis-hydroxymethyl-phenyl)-ethyl]-thiophen-3-yl}-3-ethyl-non-6-en-3-ol
(4E,6E)-7-{5-[2-(3,4-Bis-hydroxymethyl-phenyl)-ethyl]-thiophen-3-yl}-3-ethyl-nona-4,6-dien-3-ol
(3E,5E)-6-{5-[2-(3,4-Bis-hydroxymethyl-phenyl)-ethyl]-thiophen-3-yl}-1,1,1-trifluoro-2-trifluoromethyl-octa-3,5-dien-2-ol.

Selon la présente invention, les composés de formule (I) plus particulièrement préférés sont ceux pour lesquels l'une au moins, et de préférence toutes les conditions ci-dessous sont respectées :
- R₁ représente le radical -CH₃ ou -(CH₂)ₛOH,
- R₂ représente le radical -(CH₂)ₜOH,
- X-Y représente une liaison de formule (b), (c), (h) ou (g),
R₃ est choisi parmi
- une chaîne alkyle ou alkényle de 4 à 8 atomes de carbone substituée par au moins un radical hydroxyle et au moins un radical alkyle inférieur,
- ou une chaîne alkyle ou alkényle de 4 à 8 atomes de carbone substituée par au moins un radical hydroxyle, au moins un radical alkyle inférieur, et au moins un radical CF₃.

La présente invention a également pour objet les procédés de préparation des composés de formule (I).

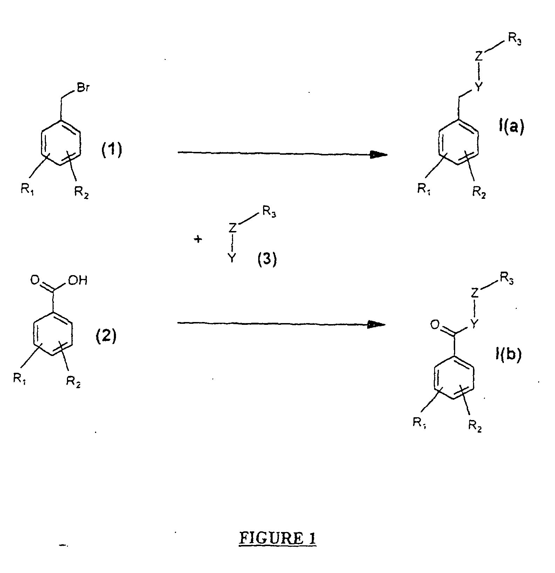

Les figures 1 à 6 représentent des schémas réactionnels qui peuvent être mis en oeuvre pour la préparation des composés selon l'invention.

Ainsi, les composés de formule **I(a)** peuvent être obtenus **(Figure 1)** par réaction d'un composé halogéné de préférence bromé **(1)** avec un dérivé **(3)** phénolique (Y=OH), thiophénolique (Y=SH), d'aniline (Y=NH-COO-tertiobutyl) en présence d'une base telle K₂CO₃ dans un solvant tel l'acétone ou la méthyléthylcétone.

Les composés de composés de formule **I(a)** peuvent être également obtenus **(Figure 1)** par réaction d'un composé halogéné de préférence bromé (1) avec le sel de sodium ou de potassium d'un dérivé **(3)** phénolique (Y=OH), thiophénolique (Y=SH), d'aniline (Y=NH-COO-tertiobutyl) dans un solvant tel le diméthylformamide (DMF).

Les composés de formule **I(b)** peuvent être obtenus **(Figure 1)** par réaction d'un dérivé benzoïque **(2)** avec un dérivé **(3)** phénolique (Y=OH), thiophénolique (Y=SH), d'aniline (Y=NH₂) en présence de carbonydiimidazole ou de dicyclohexylcarbodiimide dans un solvant tel le dichlorométhane ou le tétrahydrofuranne (THF).

Les composés de formule **I(b)** peuvent être également obtenus **(Figure 1)** par réaction d'un chlorure de benzoyle (obtenu par réaction d'un dérivé benzoïque **(2)** avec du chlorure de thionyle ou du chlorure d'oxalyle) avec un dérivé **(3)** phénolique (Y=OH), thiophénolique (Y=SH), d'aniline (Y=NH₂) en présence d'une base telle la triéthylamine dans un solvant tel le dichlorométhane ou le tétrahydrofuranne (THF).

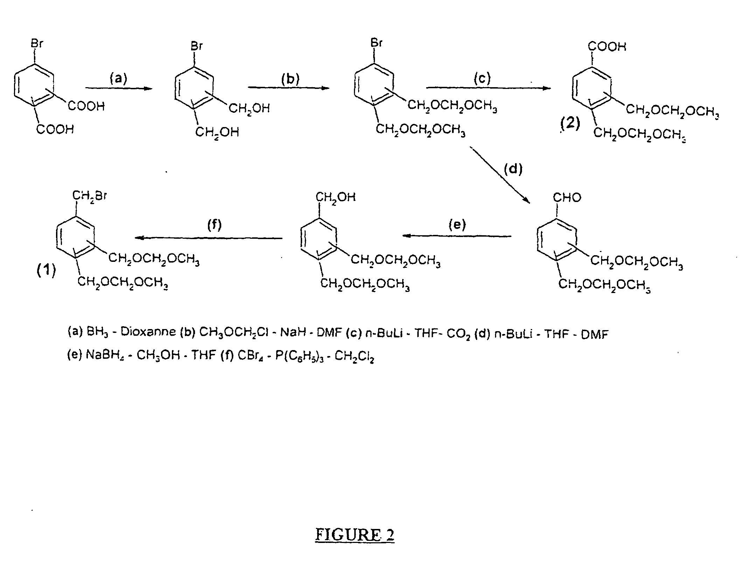

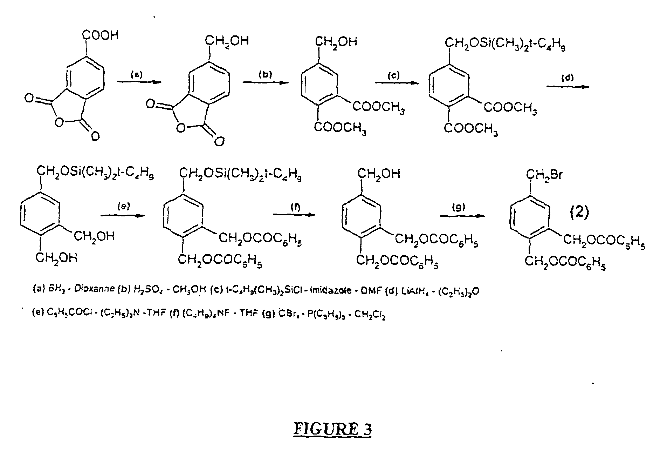

Les composés (1) et (2) peuvent être obtenus selon les schémas réactionnels présentés sur les **figure 2 et 3**. Ces méthodes de préparations des composés **(1)** et **(2)** présentent l'avantage de limiter le nombre d'étapes de préparation.

Dans la **figure 2,** (a) représente une réaction avec BH₃ dans du Dioxanne, (b) représente une réaction avec CH₃OCH₂Cl en présence d'hydrure de sodium dans un solvant diméthylformamide, (c) représente une réaction avec le n-butyllithium en présence de CO₂ dans un solvant tel le tétrahydrofuranne, (d) représente une réaction avec le n-butyllithium dans du tétrahydrofuranne suivie d'une réaction avec le diméthylformamide, (e) représente une réaction de réduction avec du borohydrure de sodium dans un solvant Méthanol-tétrahydrofuranne, (f) représente un réaction avec du tétrabromure de carbone en présence de triphénylphosphine dans un solvant dichlorométhane.

Dans la **figure 3**, (a) représente une réaction avec BH₃ dans du Dioxanne, (b) représente une réaction avec du méthanol en présence d'acide sulfurique, (c) représente une réaction avec t-C₄H₉(CH₃)₂SiCl en présence d'imidazole dans un solvant diméthylformamide, (d) représente une réaction avec LiAlH₄ dans de l'éther, (e) représente une réaction avec du chlorure de benzoyle en présence de triéthylamine dans un solvant tel le tétrahydrofuranne, (f) représente une réaction avec (C₄H₉)₄NF dans un solvant tel le tétrahydrofuranne, (g) représente un réaction avec du tétrabromure de carbone en présence de triphénylphosphine dans un solvant dichlorométhane.

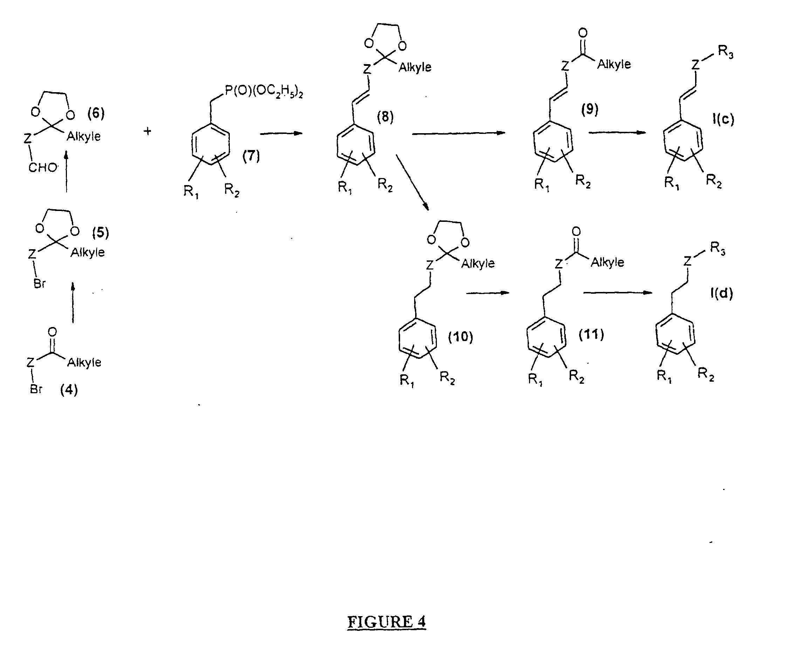

Les composés de formule **I(c)** peuvent être obtenus **(Figure 4**) par une réaction de type Horner-Emmons entre le dérivé phosphonate **(7)** (obtenu à partir du bromure de benzyle correspondant par une réaction de type Arbuzov) et le dérivé aldéhydique **(6)**. Le dérivé **(6)** peut être obtenu à partir du dérivé bromo-cétone **(4)**, tout d'abord en protégeant la fonction cétone sous forme de dioxolanne **(5)** puis par formation du dérivé lithien et réaction avec le diméthylformamide.

Les composés de formule **I(d)** peuvent être obtenus à partir des composés **(8)** par hydrogénation de la double liaison en présence de palladium sur charbon.

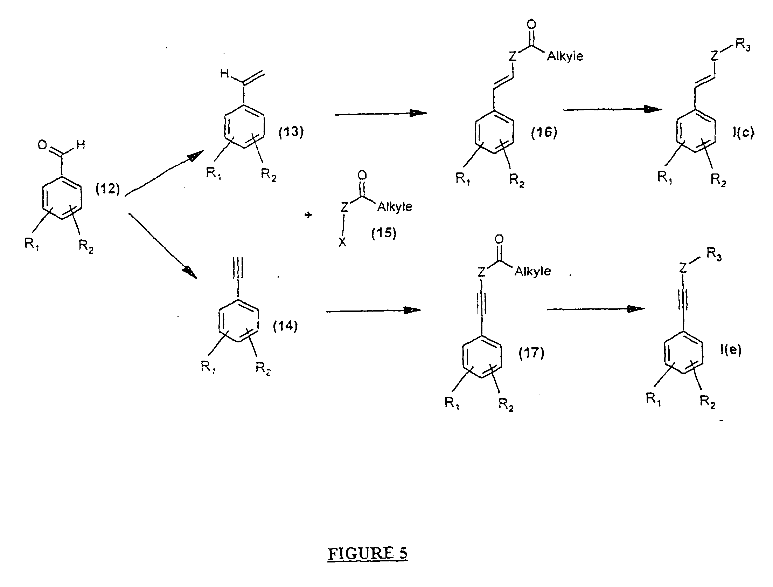

Les composés de formule **I(c)** peuvent être aussi obtenus **(Figure 5)** par une réaction de type Heck entre un dérivé éthylénique **(13)** (obtenu par réaction du benzaldéhyde **(12)** avec le bromure de méthyltriphénylphosphonium) et le dérivé triflate (X=OSO₂CF₃) ou iodé (X=I) **(15)** en présence d'un catalyseur de métaux de transition tel Pd(Cl)₂(PPh₃)₂ dans un solvant tel la triéthylamine.

Les composés de formule **I(e)** peuvent être obtenus **(Figure 5)** par réaction de type Sonogashira entre un dérivé acéthylénique **(14)** (obtenu à partir du benzaldéhyde **(12)** par une réaction de type Corey-Fuchs) et un dérivé triflate (X=OSO₂CF₃) ou Iodé (X=I) **(15)** en présence d'un catalyseur de métaux de transition tel Pd(Cl)₂(PPh₃)₂ et de CuI dans un solvant tel la triéthylamine.

La chaîne R₃ peut être introduite en utilisant par exemple les méthodes décrites dans Medicinal Research Reviews, Vol 7, N° 2, 147-171 (1987) T. KAMETANI et H. FURUYAMA, Chem. Rev. Vol 78, N° 3, 199-241 (1978) D. M. PIATAK et J. WICHA, ou dans Chem. Rev. Vol 95, N° 6,1877-1952 (1995) G. ZHU et W. H. OKAMURA.

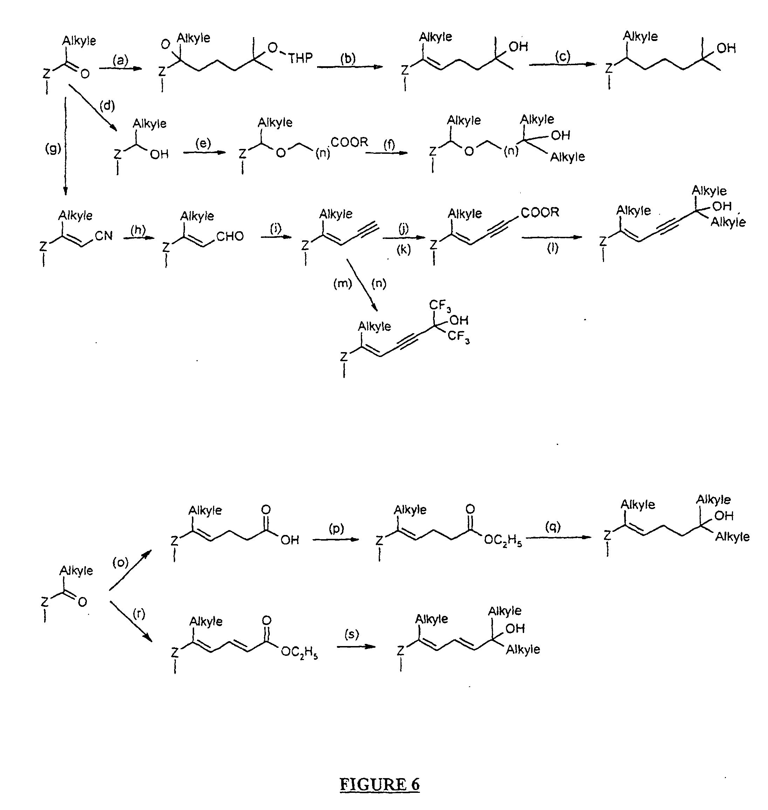

Ainsi, à titre d'exemples, quelques méthodes résumées sont données dans la figure 6 dans laquelle, (a) représente une réaction avec MgBr-CH₂-(CH₂)ₙ-C(CH₃)₂-O-tétrahydropyrane dans un solvant tel le tétrahydrofuranne, (b) représente une réaction en présence d'acide paratoluènesulfonique ou d'acide sulfurique, (c) représente une réaction d'hydrogénation en présence d'un catalyseur tel le palladium sur charbon, (d) représente une réaction de réduction avec du borohydrure de sodium dans un solvant Méthanol-tétrahydrofuranne, (e) représente une réaction avec du Br-CH₂-(CH₂)ₙ-CH₂-COOR en présence d'hydrure de potassium dans un solvant diméthylformamide, (f) représente une réaction avec MgXAlkyle, X représentant un atome d'halogène, dans un solvant tel le tétrahydrofuranne, (g) représente une réaction avec NC-CH₂-P(O)(OC₂H₅)₂ en présence d'hydrure de sodium dans un solvant tel le tétrahydrofuranne, (h) représente une réaction avec de l'hydrure de diisobutylaluminium dans un solvant tel le tétrahydrofuranne, (i) représente une réaction avec du tétrabromure de carbone en présence de triphénylphosphine dans un solvant tel le tétrahydrofuranne suivie d'une réaction avec du n-butyllithium, (j) représente une réaction avec du n-butyllithium dans un solvant telle tétrahydrofuranne, (k) représente une réaction avec le chloroformiate d'alkyle Cl-COOR, (l) représente une réaction avec MgXAlkyle, X représentant un atome d'halogène, dans un solvant tel le tétrahydrofuranne, (m) représente une réaction avec du n-butyllithium dans un solvant tel le tétrahydrofuranne, (n) représente une réaction avec CF₃-CO-CF₃,(o) représente une réaction avec HOOC-(CH₂)₃-P(C₆H₅)₃Br en présence de tertiobuthylate de potassium dans un solvant tel le tétrahydrofuranne, (p) représente une réaction avec de l'éthanol en présence d'acide sulfurique, (q) représente une réaction avec MgXAlkyle, X représentant un atome d'halogène, dans un solvant telle tétrahydrofuranne, (r) représente une réaction avec ROOC-CH=CH-CH₂-P(O)(OC₂H₅)₂ en présence de diisopropylamidure de lithium dans un solvant tel le tétrahydrofuranne, (s) représente une réaction avec un dérivé alkyllithium dans un solvant tel le tétrahydrofuranne.

Les composés de formule générale (I) présentent des propriétés biologiques analogues à celles de la vitamine D, notamment les propriétés de transactivation des éléments de réponse à la vitamine D (VDRE), telles qu'une activité agoniste ou antagoniste vis-à-vis de récepteurs de la vitamine D ou de ses dérivés. Par vitamines D ou leurs dérivés on entend par exemple les dérivés de la vitamine D₂ ou D₃ et en particulier la 1,25-dihydroxyvitamine D₃ (calcitriol).

Cette activité agoniste vis-à-vis de récepteurs de la vitamine D ou de ses dérivés peut être mise en évidence "in vitro" par des méthodes reconnues dans le domaine de l'étude de la transcription génique (Hansen et al., The Society For Investigative Dermatologie, vol.1, N°1, April 1996).

A titre d'exemple, l'activité agoniste VDR peut être testée sur la lignée, cellulaire HeLa, par cotransfection d'un vecteur d'expression du récepteur VDR humain et du plasmide rapporteur p240Hase-CAT qui contient la région - 1399 à +76 du promoteur de la 24-hydroxylase de rat, clonée en amont de la phase codante du gène de la chloramphénicol-acétyl-transférase (CAT). 18 heures après cotransfection, le produit test est ajouté dans le milieu. Après 18 heures de traitement, le dosage de l'activité CAT des lysats cellulaires est effectuée par un test Elisa. Les résultats sont exprimés en pourcentage de l'effet normalement observé avec 10⁻⁷ M de calcitriol.

L'activité agoniste peut aussi être caractérisée dans ce système de cotransfection, par la détermination de la dose nécessaire pour atteindre 50 % de l'activité maximale du produit (AC50).

Les propriétés biologiques analogues à la vitamine D peuvent être également mesurées par la capacité du produit à inhiber la prolifération des kératinocytes humaines normaux (KHN en culture). Le produit est ajouté à des KHN cultivés dans des conditions favorisant l'état prolifératif. Le produit est laissé au contact des cellules pendant cinq jours. Le nombre de cellules prolifératives est mesuré par incorporation de Bromodeoxyuridine (BRdU) dans l'ADN.

L'activité agoniste aux récepteurs de la vitamine D des composés de l'invention peut être également évaluée "in vivo" par induction de la 24-Hydroxylase chez la souris SKH. (Voorhees and al. 1997.108 : 513-518).

La présente invention a également pour objet à titre de médicament les composés de formule (I) tels que décrits ci-dessus.
Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanées telles que les kératoacanthomes,
5) pour traiter d'autres désordres dermatologiques tels que les dermatoses immunes telles le lupus érythémateux, les maladies immunes bulleuses et les maladies du collagène, telle la sclérodermie,
6) dans le traitement d'affections dermatologiques ou générales à composante immunologique,
7) pour lutter contre les troubles de la fonction sébacée tels que l'hyperséborrhée de l'acné ou la séborrhée simple,
8) dans le traitement de désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
9) pour prévenir ou traiter les troubles de la cicatrisation ou pour prévenir ou pour réparer les vergetures,
10) dans le traitement d'affections inflammatoires telles que l'arthrite,
11) dans le traitement de toute affection d'origine virale au niveau cutané ou général, telle que le syndrome de Kaposis,
12) pour traiter certains troubles ophtalmologiques, notamment les cornéopathies,
13) dans le traitement ou la prévention des états cancéreux ou précancéreux de cancers présentant ou pouvant être induits par des récepteurs de vitamine D, tels que, mais sans limitation, le cancer du sein, la leucémie, les syndromes myélodysplasiques et les lymphomes, les carcinomes des cellules de l'épithélium malpighien et les cancers gastro-intestinaux, les mélanomes, et l'ostéosarcome,
14) dans la prévention ou le traitement de l'alopécie de différentes origines, notamment l'alopécie due à la chimiothérapie ou aux rayonnements,
15) dans le traitement d'affections immunitaires, telles que les maladies auto-immunes comme le diabète sucré de type 1, la sclérose en plaques, le lupus et les affections de type lupus, l'asthme, ou la glomérulonéphrite, des dysfonctionnements sélectifs du système immunitaire tel que le SIDA, ou la prévention du rejet immun comme les rejets de greffons de rein, coeur, moelle osseuse, foie, des îlots pancréatiques, du pancréas, ou de la peau, ou la prévention de la maladie du greffon contre l'hôte,
16) dans le traitement d'affections endocriniennes, étant donné que les analogues de la vitamine D peuvent moduler la sécrétion hormonale telle que l'augmentation de la sécrétion d'insuline ou la suppression sélective de la sécrétion de l'hormone parathyroïdienne, par exemple dans l'insuffisance rénale chronique et l'hyperparathyroïdie secondaire,
17) dans le traitement d'affections caractérisées par une gestion anormale du calcium intracellulaire, et dans le traitement ou la prévention des pathologies dans lesquelles le métabolisme calcique est impliqué, telle que l'ischémie musculaire (infarctus du myocarde),
18) dans le traitement et ou la prévention des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, les troubles de la fonction parathyroïdienne,
19) dans le traitement d'affections du système cardiovasculaire telles que l'artériosclérose ou l'hypertension ainsi que le diabète non-insulino dépendant,

Dans les domaines thérapeutiques mentionnés ci-dessus, les composés selon l'invention peuvent être avantageusement employés en combinaison avec des rétinoïdes, avec des corticostéroïdes ou des oestrogènes, en association avec des anti-oxydants, avec des α-hydroxy ou α-céto acides ou leurs dérivés, avec des bloqueurs de canaux potassiques, ou encore en association avec d'autres médicaments connus pour interférer avec le système immunitaire (par exemple la cyclosporine, le FK 506, les glucocorticoïdes, les anticorps monoclonaux, les cytokines ou les facteurs de croissance ...).

Par rétinoides on entend des ligands des récepteurs RAR ou RXR, soit naturels ou soit synthétiques.

Par antiradicaux libres on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique, ascorbique, les dérivés d'acide salicylique, ainsi que leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a également pour objet une composition pharmaceutique comprenant au moins un composé de formule (I) tel que défini ci-dessus.

La présente invention a donc aussi pour objet une telle composition pharmaceutique destinée notamment au traitement des affections susmentionées.

L'administration des composés selon l'invention peut être effectuée par voie entérale, parentérale, topique ou oculaire.

Par voie entérale, les compositions pharmaceutiques peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection. Les composés selon l'invention sont généralement administrés à une dose journalière d'environ 0,001 µg/kg à 1000 µg/kg et de préférence d'environ 0,01 µg/kg à 100 µg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I) tel que défini ci-dessus à une concentration de préférence comprise entre 0,0001 et 5 % et de préférence entre 0,001 à 1% par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec les rétinoïdes, avec des corticostéroïdes, en association avec des anti-radicaux libres, avec des α-hydroxy ou α-céto acides ou leurs dérivés, ou encore avec des bloqueurs de canaux ioniques, les différents produits pris en association avec les composés de la présente invention étant tels que définis ci-dessus.

La présente invention vise donc également une composition cosmétique contenant, dans un support cosmétiquement acceptable, au moins un composé de formule I tel que défini ci-dessus. Cette composition cosmétique peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule I dans les compositions cosmétiques peut être comprise entre 0,001 et 3 % en poids par rapport au poids total de la composition.

Les compositions pharmaceutiques et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféique ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés ainsi qu'un exemple de test d'évaluation de l'activité biologique de composés de formule (I) selon l'invention.

### EXEMPLE 1

### (E)-7-[5-(3,4-Bis-hydroxyméthyl-phénoxyméthyl)-thiophen-2-yl]-3-éthyl-oct-6-en-3-ol

a) Acide 4-amino-phthalique.
   1 g (4,73 mmol) d'acide 4 nitrophtalique est dissous dans 10 ml d'éthanol anhydre. La solution est agitée à température ambiante et dégazée sous argon. 50 mg de Palladium/Charbon (5%) sont ajoutés en une seule fois et on fait buller de l'hydrogène dans la solution. Après 3 heures, la solution est filtrée sur Célite puis évaporée.
   Huile orangée. m= 820 mg. R= 96%.
   **RMN 1H** (DMSO): 3,32 (1H, s), 5,95 (1H, s), 6,49-6,53 (2H, m), 7,46-7,50 (1H, d, J= 8,8 Hz), 12,33 (2H, COOH, s).
b) 4-hydroxy-phthalate de méthyle.
   Une solution de 5 g (27,6 mmol) d'acide 4-amino-phtalique dans 50 ml d'acide sulfurique (1 M) est refroidie à 0°C. On ajoute, alors, lentement une solution de 2,27 g de nitrite de sodium dans 6 ml d'eau. Après 15 minutes à 0°C, 15 ml d'acide sulfurique concentré sont ajoutés et le mélange est porté à 100°C, sous forte agitation et pendant 1 heure. A température ambiante, le milieu réactionnel est extrait avec de l'acétate d'éthyle et lavé avec de l'eau. Après décantation, la phase organique est séchée sur sulfate de magnésium et concentrée. Le résidu est purifié sur colonne de silice (dichlorométhane 80 - méthanol 20). Il est ensuite dissous dans 100 ml de méthanol et mis à reflux avec 2 ml d'acide sulfurique. Après disparition du diacide, le méthanol est évaporé et le produit est repris par de l'acétate d'éthyle et lavé à l'eau. La phase organique est décantée, sèchée sur sulfate de sodium, évaporée.
   M= 5,2 g. R= 90%.
   **RMN 1H** (DMSO): 3,64 (3H, s), 3,67 (3H, s), 6,79-6,86 (2H, m), 7,56-7,60 (1H, d, J= 8,4 Hz), 10.51 (1H, OH, s).
c) (E)-5-(5-Bromo-thiophen-2-yl)-hex-4-enoate d'éthyle
   22,2 g (51,7 mmol) de bromure de (3-carboxypropyl)-triphénylphosphonium sont séchés sous vide pendant 1 h par chauffage à 130°C, puis ramenés à température ambiante et dissous dans 200 mL de THF anhydre. 11,5 g (102,5 mmol) de *tert*-butylate de potassium dans 100 mL de THF sont alors ajoutés lentement, puis le mélange rouge-orangé est agité pendant 15 minutes. Une solution de 7 g (34 mmol) de 1-(5-bromo-thiophen-2-yl)-éthanone dans 100 mL de THF est alors ajoutée goutte à goutte et le milieu réactionnel est agité pendant 15 heures. Après traitement par une solution saturée de chlorure d'ammonium, extraction avec de l'acétate d'éthyle, séchage et évaporation des solvants de la phase organique, le résidu obtenu est purifié par chromatographie sur colonne de silice. Un solide ocre est obtenu (PF: 62-64°C, m = 5,8 g ; R = 62%). Ce produit est alors dissous dans 100 mL d'éthanol, puis 2 mL d'acide sulfurique sont ajoutés. Le milieu réactionnel est porté à reflux et agité pendant 2 heures. Après traitement par de l'eau, le milieu est extrait avec de l'acétate d'éthyle, puis les phases organiques sont réunies, séchées et concentrées sous pression réduite. Le résidu obtenu est purifié par chromatographie sur colonne de silice (éluant heptane 95 - acétate d'éthyle 5) pour obtenir l'isomère *trans* pur sous forme d'une huile jaune (m = 3,4 g ; R = 77%).
d) (E)-7-(5-Bromo-thiophen-2-yl)-3-éthyl-oct-6-en-3-ol
   3,3 g de (E)-5-(5-bromo-thiophen-2-yl)-hex-4-enoate d'éthyle (10,9 mmol) sont dissous dans 50 mL d'éther éthylique. 22 mL d'une solution 2.0M de chlorure d'éthylmagnesium (44 mmol) sont alors ajoutés goutte à goutte et le milieu réactionnel est agité à température ambiante pendant 30 minutes. Après traitement par une solution saturée de chlorure d'ammonium, extraction avec de l'éther éthylique puis séchage et évaporation des solvants de la phase organique, le résidu obtenu est purifié par chromatographie sur colonne de silice. Une huile incolore est obtenue ( m = 2,4 g ; R = 70 %).
e) [(E)-5-(5-Bromo-thiophen-2-yl)-1,1-diéthyl-hex-4-enyloxy]-triéthylsilane
   2,2 g (6,9 mmol) de (E)-7-(5-bromo-thiophen-2-yl)-3-éthyl-oct-6-en-3-ol sont dissous dans 50 mL de dichlorométhane. 25 mg (0,2 mmol) de 4-diméthylaminopyridine et 4,8 mL de triéthylamine (34,8 mmol) sont ajoutés et le milieu réactionnel est refroidi à 0°C. 3,9 mL (17,4 mmol) de triéthylsilyltrifluorométhanesulfonate sont additionnés goutte à goutte. Après l'addition, le milieu réactionnel est ramené à température ambiante, puis traité par de l'eau et extrait avec du dichlorométhane. Après décantation, séchage et concentration sous pression réduite des phases organiques, le résidu obtenu est purifié par chromatographie sur colonne de silice. Une huile jaune est obtenue ( m = 2,3 g ; R = 97 %).
f) [5-((E)-5-Ethyl-1-méthyl-5-triéthylsilanyloxy-hept-1-enyl)-thiophen-2-yl]-méthanol
   2,8 g (6,5 mmol) de [(E)-5-(5-bromo-thiophen-2-yl)-1,1-diéthyl-hex-4-enyloxy]-triéthylsilane sont dissous dans 50 mL de THF anhydre, puis le mélange est refroidi à -78°C. 2,9 mL (7,1mmol) d'une solution de butyllithium 2,5 M sont alors ajoutés, puis le milieu réactionnel est agité pendant 15 minutes. 0,55 mL de DMF anhydre sont alors additionnés, puis le milieu réactionnel est ramené à température ambiante et. agité pendant 1 h. Après traitement par une solution saturée de chlorure d'ammonium puis extraction avec de l'acétate d'éthyle, les phases organiques sont réunies, séchées et concentrées sous pression réduite. Le résidu contenant le 5-((E)-5-éthyl-1-méthyl-5-triéthylsilanyloxy-hept-1-enyl)-thiophene-2-carbaldehyde désiré est alors dissous dans 50 mL de méthanol anhydre, puis 150 mg (3,9 mmol) de borohydrure de sodium sont ajoutés en deux portions. Après 10 minutes d'agitation le milieu est traité par une solution de chlorure d'ammonium et extrait par de l'éther éthylique. Les phases organiques sont rassemblées, séchées et concentrées sous pression réduite. Après purification par chromatographie sur colonne de silice, une huile jaune est obtenue ( m = 1,76 g ; R = 71 % ).
g) 4-[5-((E)-5-Ethyl-1-méthyl-5-triéthylsilanyloxy-hept-1-enyl)-thiophen-2-ylméthoxy]-phthalate de diméthyle
   1 g (2,6 mmol) de [5-((E)-5-éthyl-1-méthyl-5-triéthylsilanyloxy-hept-1-enyl)-thiophen-2-yl]-méthanol sont dissous dans 50 mL de dichlorométhane et refroidis à 0°C. 0,55 mL (3,9 mmol) de triéthylamine sont ajoutés, suivis de 220 µL de chlorure de méthylsulfonyle (2,9 mmol). Après 20 minutes d'agitation, le milieu réactionnel est traité par une solution de chlorure d'ammonium et extrait avec du dichlorométhane. Les phases organiques sont rassemblées, séchées et concentrées sous pression réduite. Le résidu obtenu est alors purifié par chromatographie sur colonne de silice pour conduire au produit attendu.
h) 4-[5-((E)-5-Ethyl-5-hydroxy-1-méthyl-hept-1-enyl)-thiophen-2-ylméthoxy]-phthalate de diméthyle
   750 mg (1,3 mmol) de 4-[5-((E)-5-éthyl-1-méthyl-5-triéthylsilanyloxy-hept-1-enyl)-thiophen-2-ylméthoxy]-phthalate de diméthyle sont dissous dans 30 mL de THF. 2,6 mL (2,6 mmol) d'une solution 1,0 M de fluorure de tetrabutylammonium sont additionnés et le milieu réactionnel est chauffé à 60°C pendant 3 heures. Après traitement par une solution de chlorure d'ammonium et extraction avec de l'acétate d'éthyle, les phases organiques sont rassemblées, séchées et concentrées sous pression réduite. Le résidu est purifié par chromatographie sur colonne de silice. Une huile jaune est obtenue (m = 355 mg ; R = 59%).
i) (E)-7-[5-(3,4-Bis-hydroxyméthyl-phénoxyméthyl)-thiophen-2-yl]-3-éthyl-oct-6-en-3-ol
   350 mg (0,76 mmol) de 4-[5-((E)-5-éthyl-5-hydroxy-1-méthyl-hept-1-enyl)-thiophen-2-ylméthoxy]-phthalate de diméthyle sont dissous dans 20 mL d'éther éthylique anhydre. 70 mg (1,8 mmol) d'hydrure double de lithium et d'aluminium sont additionnés et le milieu réactionnel est agité à température ambiante pendant 30 minutes. 400 µL d'eau sont alors ajoutés lentement et le milieu est filtré. Le filtrat est concentré sous pression réduite, puis le résidu obtenu est purifié par chromatographie sur colonne de silice (éluant 70 acétate d'éthyle - 30 heptane). Un solide blanc (PF: 100-2°C) est obtenu (m = 175 mg ; R = 58%).
   **RMN** ^{**1**}**H** (DMSO): 0,82 (t, 6H, *J*= 7,3 Hz), 1,42 (q, 4H, *J* = 7,4 Hz), 2,03 (s, 3H), 2,1-2,25 (m, 2H), 3,97 (s, 1H), 4,49 (d, 2H, J = 5,3 Hz), 4,58 (d, 2H, J = 5,3 Hz), 5,00 (t, 1H), 5,17 (t, 1H), 5,26 (s, 2H), 5,92 (t, 1H), 6,90-6,97 (m, 2H), 7,10 (m, 2H), 7,3 (m, 2H).

### EXEMPLE 2

### (E)-7-[4-(3,4-Bis-hydroxyméthyl-phénoxyméthyl)-thiophen-2-yl]-3-éthyl-oct-6-en-3-ol

a) (E)-5-(4-Bromo-thiophen-2-yl)-hex-4-enoate d'éthyle
   De manière analogue à l'exemple 1(c) par réaction de 18,8 g (43,9 mmol) de bromure de (3-carboxypropyl)-triphénylphosphonium avec 6 g (29,2 mmol) de 1-(4-bromo-thiophen-2-yl)-éthanone, on obtient 6 g (75%) d'acide (E)-5-(4-Bromo-thiophen-2-yl)-hex-4-enoïque, qui est transformé en 5,8 g (88%) de (E)-5-(4-Bromo-thiophen-2-yl)-hex-4-enoate d'éthyle.
b) (E)-7-(4-Bromo-thiophen-2-yl)-3-éthyl-oct-6-en-3-ol
   De manière analogue à l'exemple 1(d) à partir de 6,3 g de (E)-5-(4-bromo-thiophen-2-yl)-hex-4-enoate d'éthyle (10,9 mmol), on obtient 6,6 g (100%) de l'alcool attendu sous forme d'une huile orangée.
c) [(E)-5-(4-Bromo-thiophen-2-yl)-1,1-diéthyl-hex-4-enyloxy]-triéthylsilane
   De manière analogue à l'exemple 1(e) à partir de 6,6 g (20,3 mmol) de l'alcool précédent, on obtient 6,6 g (73%) de [(E)-5-(4-Bromo-thiophen-2-yl)-1,1-diéthyl-hex-4-enyloxy]-triéthylsilane sous forme d'une huile incolore.
d) [2-((E)-5-Ethyl-1-méthyl-5-triéthylsilanyloxy-hept-1-enyl)-thiophen-4-yl]-méthanol
   De manière analogue à l'exemple 1(f) à partir de 6,2 g (14,3 mmol) de [(E)-5-(4-Bromo-thiophen-2-yl)-1,1-diéthyl-hex-4-enyloxy]-triéthylsilane, on obient 1,7 g (29%) de [4-((E)-5-Éthyl-1-méthyl-5-triéthylsilanyloxy-hept-1-enyl)-thiophen-2-yl]-carbaldéhyde qui est réduit en présence de NaBH₄ en 1,58 g (92%) de [4-((E)-5-Éthyl-1-méthyl-5-triéthylsilanyloxy-hept-1-enyl)-thiophen-2-yl]-méthanol.
e) 4-[2-((E)-5-Ethyl-1-méthyl-5-triéthylsilanyloxy-hept-1-enyl)-thiophen-4-ylméthoxy]-phthalate de diméthyle
   De manière analogue à l'exemple 1(g) à partir de 500 mg de l'alcool précédent, on obtient 220 mg (30%) de 4-[2-((E)-5-Éthyl-1-méthyl-5-triéthylsilanyloxy-hept-1-enyl)-thiophen-4-ylméthoxy]-phthalate de diméthyle sous forme d'une huile jaune.
f) 4-[2-((E)-5-Ethyl-5-hydroxy-1-méthyl-hept-1-enyl)-thiophen-4-ylméthoxy]-phthalate de diméthyle
   De manière analogue à l'exemple 1(h) à partir de 210 mg (0,36 mmol) de 4-[2-((E)-5-Ethyl-1-méthyl-5-triéthylsilanyloxy-hept-1-enyl)-thiophen-4-ylméthoxy]-phthalate de diméthyle, on obtient 90 mg (53%) de 4-[2-((E)-5-Éthyl-5-hydroxy-1-méthyl-hept-1-enyl)-thiophen-4-ylméthoxy]-phthalate de diméthyle.
g) (E)-7-[4-(3,4-Bis-hydroxyméthyl-phénoxyméthyl)-thiophen-2-yl]-3-éthyl-oct-6-en-3-ol
   De manière analogue à l'exemple 1(i) par traitement de 4-[4-((E)-5-Ethyl-5-hydroxy-1-méthyl-hept-1-enyl)-thiophen-2-ylméthoxy]-phthalate de diméthyle (61 mg ; 0,13 mmol) par 20 mg d'hydrure double de lithiul et d'aluminium. Un solide blanc (PF: 95-7 °C) est obtenu après purification par chromatographie sur colonne de silice (m = 39 mg ; R = 73 %).
   **RMN** ^{**1**}**H** (DMSO): 0,77 (t, 6H, *J* = 7,3 Hz), 1,36 (q, 4H, *J* = 7,3 Hz), 1,94 (s, 3H), 2,0-2,15 (m, 2H), 3,89 (s, 1H), 4,41 (d, 2H, *J =* 5,3 Hz), 4,50 (d, 2H, *J*= 5,3 Hz), 4,92 (t, 1H), 5,08 (t, 1H), 5,17 (s, 2H), 5,85 (t, 1H), 6,80-6,88 (m, 2H), 7,02 (m, 2H), 7,2 (m, 2H).

### EXEMPLE 3

### (E)-7-[2-(3,4-Bis-hydroxyméthyl-phénoxyméthyl)-thiophen-4-yl]-3-éthyl-oct-6-en-3-ol

a) (E)-5-(2-Bromo-thiophen-4-yl)-hex-4-enoate d'éthyle
   De manière analogue à l'exemple 1(c) par réaction de 19,1 g (44,6 mmol) de bromure de (3-carboxypropyl)-triphénylphosphonium avec 6,1 g (29,7 mmol) de 1-(2-bromo-thiophen-4-yl)-éthanone, on obtient 5,9 g (72%) d'acide (E)-5-(2-Bromo-thiophen-4-yl)-hex-4-enoïque, qui est transformé en 4,1 g (63%) de (E)-5-(2-Bromo-thiophen-4-yl)-hex-4-enoate d'éthyle.
b) (E)-7-(2-Bromo-thiophen-4-yl)-3-éthyl-oct-6-en-3-ol
   De manière analogue à l'exemple 1(d) à partir de 4,1 g de (E)-5-(2-bromo-thiophen-4-yl)-hex-4-enoate d'éthyle (13,5 mmol), on obtient 3,2 g (75%) de l'alcool attendu sous forme d'une huile orangée.
c) [(E)-5-(2-Bromo-thiophen-4-yl)-1,1-diéthyl-hex-4-enyloxy]-triéthylsilane
   De manière analogue à l'exemple 1(e) à partir de 3,2 g (11 mmol) de l'alcool précédent, on obtient 4,2 g (96%) de [(E)-5-(2-Bromo-thiophen-4-yl)-1,1-diéthyl-hex-4-enyloxy]-triéthylsilane sous forme d'une huile incolore.
d) [4-((E)-5-Ethyl-1-méthyl-5-triéthylsilanyloxy-hept-1-enyl)-thiophen-2-yl]-méthanol
   De manière analogue à l'exemple 1(f) à partir de 4,1 g (9,5 mmol) de [(E)-5-(2-Bromo-thiophen-4-yl)-1,1-diéthyl-hex-4-enyloxy]-triéthylsilane, on obient 2 g (55%) de [4-((E)-5-Ethyl-1-méthyl-5-triéthylsilanyloxy-hept-1-enyl)-thiophen-2-yl]-carbaldéhyde qui est réduit en présence de NaBH₄ en 2 g (99%) de [4-((E)-5-Éthyl-1-méthyl-5-triéthylsilanyloxy-hept-1-enyl)-thiophen-2-yl]-méthanol.
e) 4-[4-((E)-5-Ethyl-1-méthyl-5-triéthylsilanyloxy-hept-1-enyl)-thiophen-2-ylméthoxy]-phthalate de diméthyle
   De manière analogue à l'exemple 1(g) à partir de 1 g (2,6 mmol) de l'alcool précédent, on obtient 400 mg (27%) de 4-[4-((E)-5-Ethyl-1-méthyl-5-triéthylsilanyloxy-hept-1-enyl)-thiophen-2-ylméthoxy]-phthalate de diméthyle sous forme d'une huile jaune.
f) 4-[4-((E)-5-Ethyl-5-hydroxy-1-méthyl-hept-1-enyl)-thiophen-2-ylméthoxy]-phthalate de diméthyle
   De manière analogue à l'exemple 1(h) à partir de 350 mg (0,6 mmol) de 4-[4-((E)-5-Ethyl-1-méthyl-5-triéthylsilanyloxy-hept-1-enyl)-thiophen-2-ylméthoxy]-phthalate de diméthyle, on obtient 190 mg (68%) de 4-[4-((E)-5-Éthyl-5-hydroxy-1-méthyl-hept-1-enyl)-thiophen-2-ylméthoxy]-phthalate de diméthyle.
g) (E)-7-[2-(3,4-Bis-hydroxyméthyl-phénoxyméthyl)-thiophen-4-yl]-3-éthyl-oct-6-en-3-ol
   De manière analogue à l'exemple 1(i), par traitement de 4-[5-((E)-5-Ethyl-5-hydroxy-1-méthyl-hept-1-enyl)-thiophen-3-ylméthoxy]-phthalate de diméthyle (180 mg ; 0,39 mmol) par 36 mg d'hydrure double de lithium et d'aluminium (0,9 mmol). Une huile incolore est obtenue après purification par chromatographie sur colonne de silice (m = 115 mg ; R = 73 %).
   **RMN** ^{**1**}**H** (DMSO): 0,85 (t, 6H, *J*= 7,3 Hz), 1,43 (q, 4H, *J*= 7,4 Hz), 1,98 (s, 3H), 2,13-2,20 (m, 2H), 3,95 (s, 1H), 4,49 (d, 2H, *J* = 5,3 Hz), 4,58 (d, 2H, *J* = 5,3 Hz), 5,00 (t, 1H, *J* = 5,4 Hz), 5,16 (t, 1H, *J* = 5,5 Hz), 5,27 (s, 2H), 5,98 (t, 1H), 6,92 (dd, 1H, *J*_{*1*}*=* 2,6 Hz, *J*_{*2*} = 8,3 Hz), 7,10 (d, 1H, *J*= 2,5 Hz), 7,28-7,35 (m, 2H), 7,44 (s, 1H).

### EXEMPLE 4

### (E)-7-[6-(3,4-Bis-hydroxyméthyl-phénoxyméthyl)-pyridin-2-yl]-3-éthyl-non-6-en-3-ol

a) (E)-5-(6-Bromo-pyridin-2-yl)-hept-4-enoate d'éthyle
   De manière analogue à l'exemple 1(c) par réaction de 29,8 g (69,3 mmol) de bromure de (3-carboxypropyl)-triphénylphosphonium avec 9,9 g (46,2 mmol) de 1-(6-bromo- pyridin-2-yl)-propanone, on obtient 8,9 g (68%) d'acide (E)-5-(6-Bromo- pyridin-2-yl)-hept-4-enoïque, qui est transformé en 7,8 g (80%) de (E)-5-(6-Bromo- pyridin-2-yl)-hept-4-enoate d'éthyle.
b) (E)-7-(6-Bromo- pyridin-2-yl)-3-éthyl-non-6-en-3-ol
   De manière analogue à l'exemple 1(d) à partir de 7,4 g de (E)-5-(6-bromo- pyridin-2-yl)-hept-4-enoate d'éthyle (23,7 mmol), on obtient 7,3 g (94%) de l'alcool attendu sous forme d'une huile orangée.
c) [(E)-5-(6-Bromo- pyridin-2-yl)-1,1-diéthyl-hept-4-enyloxy]-triéthylsilane
   De manière analogue à l'exemple 1(e) à partir de 7,5 g (23 mmol) de l'alcool précédent, on obtient 9,8 g (97%) de [(E)-5-(6-Bromo- pyridin-2-yl)-1,1-diéthyl-hept-4-enyloxy]-triéthylsilane sous forme d'une huile incolore.
d) [6-((E)-1,5-diéthyl-5-triéthylsilanyloxy-hept-1-enyl)-pyridin-2-yl]-méthanol
   De manière analogue à l'exemple 1(f) à partir de 8,8 g (20 mmol) de [(E)-5-(6-Bromo-pyridin-2-yl)-1,1-diéthyl-hept-4-enyloxy]-triéthylsilane, on obient 5,8 g (75%) de [6-((E)- 1,5-diéthyl -5-triéthylsilanyloxy-hept-1-enyl)- pyridin-2-yl]-carbaldéhyde qui est réduit en présence de NaBH₄ en 5,8 g (99%) de [6-((E)- 1,5-diéthyl-5-triéthylsilanyloxy-hept-1-enyl)-pyridin-2-yl]-méthanol.
e) 4-[6-((E)-1,5-diéthyl -5-triéthylsilanyloxy-hept-1-enyl)- pyridin-2-ylméthoxy]-phthalate de diméthyle
   De manière analogue à l'exemple 1(g) à partir de 1 g (2,55 mmol) de l'alcool précédent; on obtient 1,2 g (81%) de 4-[6-((E)-1,5-diéthyl -5-triéthylsilanyloxy-hept-1-enyl)- pyridin-2-ylméthoxy]-phthalate de diméthyle sous forme d'une huile jaune.
f) 4-[6-((E)-1,5-diéthyl -5-hydroxy--hept-1-enyl)- pyridin-2-ylméthoxy]-phthalate de diméthyle
   De manière analogue à l'exemple 1(h) à partir de 1,1 g (1,88 mmol) de 4-[6-((E)-1,5-diéthyl-5-triéthylsilanyloxy-hept-1-enyl)- pyridin-2-ylméthoxy]-phthalate de diméthyle, on obtient 740 mg (84%) de 4-[6-((E)-1,5-diéthyl -5-hydroxy-hept-1-enyl)- pyridin-2-ylméthoxy]-phthalate de diméthyle.
g) (E)-7-[6-(3,4-Bis-hydroxyméthyl-phénoxyméthyl)-pyridin-2-yl]-3-éthyl-non-6-en-3-ol]-3-éthyl-oct-6-en-3-ol
   De manière analogue à l'exemple 1(i), par traitement de 4-[6-((E)-1,5-diéthyl-5-hydroxy-hept-1-enyl)-pyridin-2-ylméthoxy]-phthalate de diméthyle (740 mg ; 1,57 mmol) par 145 mg d'hydrure double de lithium et d'aluminium (3,8 mmol). Une huile incolore est obtenue après purification par chromatographie sur colonne de silice (m = 515 mg ; R = 79%).
   **RMN** ^{**1**}**H** (CDCl₃): 0,88 (t, 6H, *J*= 7,4 Hz), 1,04 (t, 3H, *J*= 7,5 Hz), 1,48-1,64 (m, 6H), 1,7 (bs, 1H), 2,24-2,33 (m, 2H), 2,65 (q, 2H, *J*= 7,5 Hz), 3,1 (bs, 1H), 3,3 (bs, 1H), 4,65 (s, 2H), 4,68 (s, 2H), 5,16 (s, 2H), 6,24 (t, 1H, J = 7,3 Hz), 6,88 (dd, 1H, *J*_{*1*} = 2,6 Hz, *J*_{*2*} = 8,3 Hz)), 7,03 (d, 1H, *J=* 2,6 Hz), 7,20-7,32 (m, 3H), 7,62 (t, 1H, *J=* 7,8 Hz).

### EXEMPLE 5

### (E)-7-[5-(3,4-Bis-hydroxyméthyl-phén(oxyméthyl)-pyridin-3-yl]-3-éthyl-non-6-en-3-ol

(a) 5-Bromo-N-méthoxy-N-méthyl-nicotinamide
   40 g (198 mmol) d'acide 5-bromonicotinique sont dissous dans 300 ml de THF et traités par 19 ml de chlorure d'oxalyle à 0°C. 66 ml (475 mmol) de triéthylamine sont ensuite ajoutés, suivi par 23,2 g (237 mmol) de chlorydrate de N-O-diméthylhydroxylamine. Après agitation à température ambiante pendant 4 heures, le milieu réactionnel est versé dans l'eau, extrait avec de l'acétate d'éthyle. La phase organique est décantée, séchée sur sulfate de magnésium, évaporée. On recueille 45,1 g (93%) du produit attendu sous forme d'une huile marron clair.
(b) 1-(5-bromo-pyridin-3yl)-propanone
   44 g (180 mmol) de 5-Bromo-N-méthoxy-N-méthyl-nicotinamide sont dissous dans 200 ml de THF et 60 ml (180 mmol) d'une solution de chlorure d'éthylmagnésium (3M dans l'éther éthylique) sont ajoutés goutte à goutte. Le milieu réactionnel est agité à température ambiante pendant une heure, versé dans l'eau, extrait avec de l'acétate d'éthyle. La phase organique est décantée, séchée sur sulfate de magnésium, évaporée. Le résidu obtenu est purifié par chromatographie sur colonne de silice élué avec un mélange d'heptane et d'acétate d'éthyle (70-30). On recueille 4 g (11%) de 1-(5-bromo-pyridin-3yl)-prppanone.
c) (E)-5-(5-Bromo-pyridin-3-yl)-hept-4-enoate d'éthyle
   De manière analogue à l'exemple 1(c) par réaction de 12 g (28 mmol) de bromure de (3-carboxypropyl)-triphénylphosphonium avec 4 g (18,6 mmol) de 1-(5-bromo- pyridin-3-yl)-propanone, on obtient 4,45 g (84%) d'acide (E)-5-(5-Bromo- pyridin-3-yl)-hept-4-enoïque, qui est transformé en 3,6 g (74%) de (E)-5-(5-Bromo- pyridin-3-yl)-hept-4-enoate d'éthyle.
d) (E)-7-(5-Bromo- pyridin-3-yl)-3-éthyl-non-6-en-3-ol
   De manière analogue à l'exemple 1(d) à partir de 2,6 g de (E)-5-(5-bromo- pyridin-3-yl)-hept-4-enoate d'éthyle (8,3 mmol), on obtient 2,3 g (85%) de l'alcool attendu sous forme d'une huile orangée.
e) [(E)-5-(5-Bromo- pyridin-3-yl)-1,1-diéthyl-hept-4-enyloxy]-triéthylsilane
   De manière analogue à l'exemple 1(e) à partir de 2,3 g (7 mmol) de l'alcool précédent, on obtient 2,9 g (93%) de [(E)-5-(5-Bromo- pyridin-3-yl)-1,1-diéthyl-hept-4-enyloxy]-triéthylsilane sous forme d'une huile incolore.
f) [5-((E)-1,5-diéthyl-5-triéthylsilanyloxy-hept-1-enyl)-pyridin-3-yl]-méthanol
   De manière analogue à l'exemple 1(f) à partir de 2,9 g (6,6 mmol) de [(E)-5-(5-Bromo-pyridin-3-yl)-1,1-diéthyl-hept-4-enyloxy]-triéthylsilane, on obient 775 mg (30%) de [5-((E)-1,5-diéthyl -5-triéthylsilanyloxy-hept-1-enyl)- pyridin-3-yl]-carbaldéhyde qui est réduit en présence de NaBH₄ en 774 mg (99%) de [5-((E)-1,5-diéthyl -5-triéthylsilanyloxy-hept-1-enyl)- pyridin-3-yl]-méthanol.
g) 4-[5-((E)-1,5-diéthyl -5-triéthylsilanyloxy-hept-1-enyl)- pyridin-3-ylméthoxy]-phthalate de diméthyle
   De manière analogue à l'exemple 1(g) à partir de 750 mg (1,9 mmol) de l'alcool précédent, on obtient 140 mg (13%) de 4-[5-((E)-1,5-diéthyl -5-triéthylsilanyloxy-hept-1-enyl)- pyridin-3-ylméthoxy]-phthalate de diméthyle sous forme d'une huile jaune.
h) 4-[5-((E)- 1,5-diéthyl -5-hydroxy-hept-1-enyl)- pyridin-3-ylméthoxy]-phthalate de diméthyle
   De manière analogue à l'exemple 1(h) à partir de 100 mg (0,17 mmol) de 4-[5-((E)-1,5-diéthyl- 5-triéthylsilanyloxy-hept-1-enyl)- pyridin-3-ylméthoxy]-phthalate de diméthyle, on obtient 66 mg (82%) de 4-[5-((E)-1,5-diéthyl-5-hydroxy-hept-1-enyl)- pyridin-3-ylméthoxy]-phthalate de diméthyle.
i) (E)-7-[5-(3,4-Bis-hydroxyméthyl-phénoxyméthyl)-pyridin-3-yl]-3-éthyl-non-6-en-3-ol
   De manière analogue à l'exemple 1(i), par traitement de 60 mg (0,13 mmol) de 4-[5-((E)-1,5-Diéthyl-5-hydroxy-hept-1-enyl)-pyridin-3-ylméthoxy]-phthalate de diméthyle par 12 mg d'hydrure double de lithium et d'aluminium . Une huile incolore est obtenue après purification par chromatographie sur colonne de silice (m = 25 mg ; R = 47%).
   **RMN** ^{**1**}**H** (CDCl₃): 0,90 (t, 6H, *J =* 7,4 Hz), 0,98 (t, 3H, *J =* 7,4 Hz), 1,48-1,60 (m, 6H), 2,20-2,26 (m, 2H), 2,52 (q, 2H, *J*= 7,4 Hz), 4,69 (s, 2H), 4,71 (s, 2H), 5,04 (s, 2H), 5,69 (t, 1H, J = 7,3 Hz), 6,87 (dd, 1H, *J*_{*1*} = 2,7 Hz, *J*_{*2*} = 8,3 Hz)), 7,02 (d, 1H, *J*= 2,6 Hz), 7,25-7,28 (m, 1H), 7,72 (m, 2H), 8,4 (bs, 1H), 8,5 ( bs, 1H).

### EXEMPLE 6

### (E)-7-[5-(3,4-Bis-hydroxyméthyl-phénoxyméthyl)-thiophen-2-yl]-3-éthyl-non-6-en-3-ol

a) 2-Éthyl-2-thiophen-2-yl-[1,3]dioxolane
   30 g (214 mmol) de 1-thiophen-2-yl-propan-1-one sont dissous dans 120 ml d'éthylene glycol. 93g (856 mmol) de chlorure de triméthylsilyl sont alors ajoutés. Le milieu reactionnel est agité à température ambiante pendant 24 heures. Après traitement par de l'eau, le milieu est extrait avec de l'acétate d'éthyle, puis les phases organiques sont réunies, séchées et concentrées sous pression réduite. Le résidu obtenu est purifié par chromatographie sur colonne de silice (éluant heptane 90 - acétate d'éthyle 10).Une huile jaune est obtenue (m = 15 g ; R = 38%).
b) 5-(2-Éthyl-[1,3]dioxolan-2-yl)-thiophene-2-carbaldehyde
   15g (81,4 mmol) de 2-éthyl-2-thiophen-2-yl-[1,3]dioxolane sont dissous dans 300 ml de THF anhydre puis le mélange est refroidi à -78°C. 53 ml (89 mmol) d'une solution de *tert*-butyllithium 1,7M sont alors ajoutés puis le milieu réactionnel est agité pendant 1heure. 10 ml (120 mmol) de DMF anhydre sont alors ajoutés et le milieu est agité pendant 1 heure. A -78°C , traitement du milieu réactionnel par une solution d'acide chlorhydrique 1N puis extraction avec de l'acétate d'éthyle, les phases organiques sont réunies, séchées et concentrées sous pression réduite. Le résidu obtenu est purifié par chromatographie sur colonne de silice. Une huile jaune est obtenue (m=19 g ; R=100%).
c) [5-(2-Ethyl-[1,3]dioxolan-2-yl)-thiophen-2-yl]-méthanol
   9,5g (44,7 mmol) 5-(2-éthyl-[1,3]dioxolan-2-yl)-thiophene-2-carbaldehyde sont dissous dans 100 ml de méthanol anhydre puis, le mélange est refroidi à 0°C.
   2,2g (58,1 mmol) de borohydrure de sodium sont alors ajoutés par petites fractions. Après 1 heure d'agitation le milieu est traité par une solution de chlorure d'ammonium et extrait par de l'acétate d'éthyle. Les phases organiques sont rassemblées, séchées et concentrées sous pression réduite. Après purification par chromatographie sur colonne de silice, une huile jaune est obtenue (m = 9,25 g ; R = 96 %).
d) Méthanesulfonate de 5-(2-éthyl-[1,3]dioxolan-2-yl)-thiophen-2-ylméthyl
   9,25 g (43,2 mmol) de [5-(2-éthyl-[1,3]dioxolan-2-yl)-thiophen-2-yl]-méthanol sont dissous dans 500 ml de dichlorométhane et refroidis à 0°C. 9,0 ml (64,7 mmol) de triéthylamine sont ajoutés, suivis de 3,5ml de chlorure de méthylsulfonate (45,3 mmol). Après 1 heure d'agitation, le milieu réactionnel est traité par une solution de chlorure d'ammonium et extrait avec du dichlorométhane. Les phases organiques sont rassemblées, séchées et concentrées sous pression réduite. Une huile marron est obtenue (m=9,28g ; R=75%).
e) 4-[5-(2-Ethyl-[1,3]dioxolan-2-yl)-thiophen-2-ylméthoxy]-phthalate de diméthyle
   9,25g (43,2 mmol) de méthanesulfonate de 5-(2-éthyl-[1,3]dioxolan-2-yl)-thiophen-2-ylméthyl sont dissous dans 100 ml de 2-butanone, et 9,07g (43,2 mmol) de 4-hydroxyphthalate de diméthyle (préparé à l'exemple 1(b)), 6 g de carbonate de potassium (43,2 mmol) et 20 mg d'iodure de sodium sont ajoutés. Le mélange est chauffé à reflux pendant 12 heures, refroidi et filtré. Le filtrat est concentré sous pression réduite, puis purifié par chromatographie sur colonne de silice. Une huile jaune est obtenue (m = 12,70g ; R = 73%).
f) {5-[5-(2-Ethyl-[1,3]dioxolan-2-yl)-thiophen-2-ylméthoxy]-2-hydroxyméthyl-phényl}-méthanol
   12,7 g (31,5 mmol) de 4-[5-(2-éthyl-[1,3]dioxolan-2-yl)-thiophen-2-ylméthoxy]-phthalate de diméthyle sont dissous dans 500 ml d'éther éthylique anhydre. 2,87 g (75,7 mmol) d'hydrure double de lithium et d'aluminium sont additionnés et le milieu réactionnel est agité à 0°C pendant 1 heure. 2,9 ml d'eau sont alors ajoutés lentement puis 2,9 ml de soude 15% aqueuse puis 8,7 ml d'eau sont également lentement ajoutés. Apres 20 minutes d'agitation le milieu est alors filtré. Le filtrat est concentré sous pression réduite, puis le résidu obtenu est purifié par chromatographie sur colonne de silice. Un solide blanc (PF: 78-80 °C) est obtenu (m = 8 g ; R = 72%).
g) 1-(5-(3,4-Bis-hydroxyméthyl-phénoxyméthyl)- thiophen-2-yl]-propan-1-one
   8 g (22,8 mmol) de {5-[5-(2-éthyl-[1,3]dioxolan-2-yl)-thiophen-2-ylméthoxy]-2-hydroxyméthyl-phényl}-méthanol sont dissous dans 75 ml d'eau et 75 ml d'acetone. 1g d'acide para-toluenesulfonique est alors ajouté et le milieu réactionnel est porté à reflux pendant 1heure. Le milieu réactionnel est traité par une solution de bicarbonate de sodium et extrait avec de l'acétate d'éthyle. Les phases organiques sont rassemblées, laveés avec de l'eau, séchées et concentrées sous pression réduite. Une poudre blanche (Pf=123°C) est obtenue (m = 6,9 g ; R=100%).
h) 1-{5-[3,4-Bis-(*tert*-butyl-diméthyl-silanyloxyméthyl)-phénoxyméthyl]- thiophen-2-yl}-propan-1-one
   6,9 g (22,8 mmol) de 1-[5-(3,4-bis-hydroxyméthyl-phénoxyméthyl)- thiophen-2-yl]-propan-1-one sont dissous dans 70 ml de DMF anhydre. 4,3g (63,8 mmol) d'imidazole et 7,5 g (50 mmol) de *tert*-butyldiméthylchlorosilane sont alors ajoutés par petites fractions. Le milieu réactionnel est agité à température ambiante pendant 12 heures. Ce dernier est traité par de l'eau et extrait avec de l'acétate d'éthyle. Les phases organiques sont rassemblées, séchées et concentrées sous pression réduite puis le résidu obtenu est purifié par chromatographie sur colonne de silice. Une huile incolore est obtenue (m = 11,5 g ; R = 100%).
i) Acide (E)-5-{5-[3,4-bis-(*tert*-butyl-diméthyl-silanyloxyméthyl)-phénoxyméthyl]- thiophen-2-yl}-hept-4-enoique
   6,7 g (15,6 mmol) de bromure de (3-carboxypropyl)-triphénylphosphonium sont séchés sous vide pendant 1 h par chauffage à 130°C, puis ramenés à température ambiante et dissous dans 70 ml de THF anhydre. 3,5 g (31,2 mmol) de *tert*-butylate de potassium sont alors ajoutés lentement, puis le mélange rouge-orangé est agité pendant 15 minutes. Une solution de 5,7 g (10,4 mmol) de 1-{5-[3,4-Bis-(*tert*-butyl-diméthyl-silanyloxyméthyl)-phénoxyméthyl]-thiophen-2-yl}-propan-1-one
   dans 70 ml de THF est alors ajoutée goutte à goutte et le milieu réactionnel est agité pendant 4 heures. Après traitement par une solution saturée de chlorure d'ammonium, extraction avec de l'acétate d'éthyle, séchage et évaporation des solvants de la phase organique, le résidu obtenu est purifié par chromatographie sur colonne de silice. Une huile jaune est obtenue (m = 4,3 g ; R = 66%).
j) (E)-5-{5-[3,4-Bis-(*tert*-butyl-diméthyl-silanyloxyméthyl)-phénoxyméthylthiophen-2-yl}-hept-4-enoate de méthyle
   4,3 g (7,1 mmol) d'acide (E)-5-{5-[3,4-bis-(*tert*-butyl-diméthyl-silanyloxyméthyl)-phénoxyméthyl]-thiophen-2-yl}-hept-4-enoique sont dissous dans 70 ml de 2-butanone, 1,1g de carbonate de potassium (7,8 mmol) et 2,2 ml de iodométhane (35 mmol) sont ajoutés. Le mélange est chauffé à reflux pendant 12 heures, refroidi et filtré. Le filtrat est concentré sous pression réduite, puis purifié par chromatographie sur colonne de silice. Une huile jaune est obtenue (m = 4,34 g ; R = 98%).
k) (E)-7-{5-[3,4-Bis-(*tert*-butyl-diméthyl-silanyloxyméthyl)-phénoxyméthyl]- thiophen-2-yl}-3-éthyl-non-6-en-3-ol
   1,3 g (2,3 mmol) de (E)-5-{5-[3,4-bis-(*tert*-butyl-diméthyl-silanyloxyméthyl)-phénoxyméthyl]- thiophen-2-yl}-hept-4-enoate de méthyle sont dissous dans 20 ml d'éther éthylique. 2,2 ml d'une solution 3.0 M de bromure d'éthylmagnesium (6,5 mmol) sont alors ajoutés goutte à goutte et le milieu réactionnel est agité à température ambiante pendant 2 heures. Après traitement par une solution saturée de chlorure d'ammonium, extraction avec de l'acétate d'éthyle puis séchage et évaporation des solvants de la phase organique, le résidu obtenu est purifié par chromatographie sur colonne de silice. Après séparation de l'isomère Z de l'isomère E, une huile incolore est obtenue (m = 440 mg ; R =29 %).
1) (E)-7-[5-(3,4-Bis-hydroxyméthyl-phénoxyméthyl)-thiophen-2-yl]-3-éthyl-non-6-en-3-ol
   440 mg (0,7 mmol) de (E)-7-{4-[3,4-bis-(*tert*-butyl-diméthyl-silanyloxyméthyl)-phénoxyméthyl]-cyclopenta-1,3-dienyl}-3-éthyl-non-6-en-3-ol sont dissous dans 20 ml de THF. 2,0 ml (2,0 mmol) d'une solution 1,0 M de fluorure de tetrabutylammonium sont additionnés et le milieu réactionnel est agité à température ambiante pendant 1 heure. Après traitement par une solution de chlorure d'ammonium et extraction avec de l'acétate d'éthyle, les phases organiques sont rassemblées, séchées et concentrées sous pression réduite. Le résidu est purifié par chromatographie sur colonne de silice (éluant 80 acétate d'éthyl-20 heptane) Une poudre blanche (PF :82°C) est obtenue (m = 248 mg ; R = 89%).
   **RMN** ^{**1**}**H** (DMSO): 0,82 (t, 6H, J = 7,5 Hz); 1,07 (t, 3H, J = 7,4 Hz); 1,36-1,46 (m, 6H); 2,03 (s, 1H); 2,10-2,20 (m, 2H); 2,42-2,51 (m, 2H); 4,47 (d, 2H, J = 4,9 Hz); 4,56 (d, 2H, J = 4,9 Hz); 4,99 (t, 1H, J = 4,9 Hz); 5,15 (t, 1H, J = 4,9 Hz); 5,23 (s, 2H); 5,82 (t, 1H, J = 7,3 Hz); 6,87-6,96 (m, 2H); 7,08 (d, 2H, J = 2,8 Hz); 7,28 (d, 1H, 8,3 Hz).

### EXEMPLE 7

### (4E,6E)-7-[5-(3,4-Bis-hydroxyméthyl-phénoxyméthyl)-thiophen-2-yl]-3-éthyl-nona-4,6-dien-3-ol

a) (E)-3-{5-[3,4-Bis-(*tert*-butyl-diméthyl-silanyloxyméthyl)-phénoxyméthyl]-thiophen-2-yl}-pent-2-enoate d'éthyle
   4,1 ml (20 mmol) de phosphonoacetate de triéthyle sont dissous dans 100 ml de THF anhydre, puis NaH (0,8 g, 20 mmol) est additionné par fractions. Après 30 minutes d'agitation, une solution de 5,75 g (10,4 mmol) de 1-{5-[3,4-Bis-(*tert*-butyl-diméthyl-silanyloxyméthyl)-phénoxyméthyl]- thiophen-2-yl}-propan-1-one (préparé à l'exemple 6(h)) dans 50 ml de THF anhydre est additionnée goutte à goutte, et le milieu réactionnel est chauffé pendant 14 heures à 50°C. Après le traitement usuel et chromatographie sur gel de silice (éluant heptane 90 - acétate d'éthyle 10), le produit désiré est séparé de son isomère Z et obtenu sous forme d'huile incolore (m = 3,18 g ; R = 50%).
b) (E)-3-{5-[3,4-Bis-(*tert*-butyl-diméthyl-silanyloxyméthyl)-phénoxyméthyl]-thiophen-2-yl}-pent-2-en-1-ol
   3,18 g (5,2 mmol) de (E)-3-{5-[3,4-bis-(*tert*-butyl-diméthyl-silanyloxyméthyl)-phénoxyméthyl]-thiophen-2-yl}-pent-2-enoate d'éthyle en solution dans 20 ml d'éther éthylique sont ajoutés goutte à goutte à une suspension d'hydrure double de lithium et d'aluminium ( 0,24 g, 6,3 mmol) dans 50 ml d'éther. Le milieu est agité à température ambiante pendant 2 heures, puis traité successivement par 240 µL d'eau, 240 µL de soude 15% et 720 µL d'eau. Le milieu est alors filtré et le filtrat concentré sous pression réduite. Le produit désiré (m = 2,92 g ; R = 93%) est obtenu sous forme d'huile incolore.
c) (E)-3-{5-[3,4-Bis-(*tert*-butyl-diméthyl-silanyloxyméthyl)-phénoxyméthyl]-thiophen-2-yl}-pent-2-enal
   2,74 g (5,1 mmol) de (E)-3-{5-[3,4-bis-(*tert*-butyl-diméthyl-silanyloxyméthyl)-phénoxyméthyl]-thiophen-2-yl}-pent-2-en-1-ol sont dissous dans 80 ml de dichlorométhane, et placés sous atmosphère d'azote. 4,45 g (51 mmol) de dioxyde de manganèse sont additionnés, et le milieu est agité pendant 14 heures. Après filtration puis évaporation, une huile jaune est obtenue. Le produit brut (2,9 g) est l'aldéhyde attendu, obtenu avec un rendement quantitatif.
d) (2E,4E)-5-{5-[3,4-Bis-(*tert*-butyl-diméthyl-silanyloxyméthyl)-phénoxyméthyl]-thiophen-2-yl}-hepta-2,4-dienoate d'éthyle
   De manière analogue à l'exemple 7(a), par réaction de 2,9 g (5,1 mmol) de (E)-3-{5-[3,4-Bis-(*tert*-butyl-diméthyl-silanyloxyméthyl)-phénoxyméthyl]-thiophen-2-yl}-pent-2-enal avec 1,5 ml (7,6 mmol) de phosphonoacétate de triéthyle. Un isomère unique est obtenu: (2E,4E)-5-{5-[3,4-Bis-(*tert*-butyl-diméthyl-silanyloxyméthyl)-phénoxyméthyl]-thiophen-2-yl}-hepta-2,4-dienoate d'éthyle est isolé sous forme d'huile jaune (m = 2,17g ;R = 67%).
e) (4E,6E)-7-{5-[3,4-Bis-(*tert*-butyl-diméthyl-silanyloxyméthyl)-phénoxyméthyl]-thiophen-2-yl}-3-éthyl-nona-4,6-dien-3-ol
   Une solution d'éthyl lithium (1,5 M) est préparée par lente addition d'une solution de bromure d'éthyle (11,2 ml, 150 mmol) dans 50 ml de pentane à une suspension de lithium (2,75 g, 400 mmol) dans 50 ml de pentane à 40°C, puis agitation à 40°C pendant 12 heures. Après refroidissement à température ambiante, 23 ml (34 mmol) de cette solution sont ajoutés goutte à goutte à une solution à 0°C de (2E,4E)-5-{5-[3,4-bis-(*tert*-butyl-diméthyl-silanyloxyméthyl)-phénoxyméthyl]-thiophen-2-yl}-hepta-2,4-dienoate d'éthyle dans 50 ml de THF. Le milieu devient rouge et est agité pendant 2 heures à 0°C, puis traité par addition d'une solution saturée de chlorure d'ammonium. Après le traitement usuel, le résidu est purifié par chromatographie sur gel de silice (éluant heptane 95 - acétate d'éthyle 5). Le produit désiré est obtenu sous forme d'huile jaune (m = 0,85 g, R = 38%).
f) (4E,6E)-7-[5-(3,4-Bis-hydroxyméthyl-phénoxyméthyl)-thiophen-2-yl]-3-éthyl-nona-4,6-dien-3-ol
   850 mg (1,3 mmol) de (4E,6E)-7-{5-[3,4- bis-(*tert*-butyl-diméthyl-silanyloxyméthyl)-phénoxyméthyl]-thiophen-2-yl}-3-éthyl-nona-4,6-dien-3-ol sont dissous dans 20 ml de THF anhydre. 3,9 ml (3,9 mmol) d'une solution 1 M de fluorure de tetrabutylammonium sont alors ajoutés, et le milieu est agité pendant 4 heures. Après le traitement usuel, le résidu est purifié par chromatographie sur gel de silice ( éluant heptane 2 - acétate d'éthyle 8). (4E,6E)-7-[5-(3,4-Bis-hydroxyméthyl-phénoxyméthyl)-thiophen-2-yl]-3-éthyl-nona-4,6-dien-3-ol est obtenu sous forme d'huile jaune (m = 340 mg ; R = 63% ).
   **RMN** ^{**1**}**H** (DMSO): 0,74 (t, 6H, J = 7,5 Hz); 1,13 (t, 3H, J = 7,4 Hz); 1,36-1,45 (m, 4H); 2,26 (t, 1H, J = 6,9 Hz); 2,46-2,53 (m, 2H); 4,38 (d, 2H, J = 4,8 Hz); 4,47 (d, 2H, J = 4,8 Hz); 4,90 (t, 1H, J = 4,8 Hz); 5,05 (t, 1H, J = 4,8 Hz); 5,16 (s, 2H); 5,76 (d, 1H, J = 13,8 Hz); 6,36-6,47 (m, 2H); 6,79-6,83 (m, 1H); 6,98-7,04 (m, 3H); 7,20 (d, 1H, J = 8,3 Hz).

### EXEMPLE 8

### (E)-7-{5-[2-(3,4-Bis-hydroxyméthyl-phényl)-éthyl]-thiophen-2-yl}-3-éthyl-non-6-en-3-ol

a) 4-{(E)-2-[5-(2-Ethyl-[1,3]dioxolan-2-yl)-thiophen-2-yl]-vinyl}-phthalate de diméthyle
   18,5 g (53,7 mmol) de 4-(diéthoxy-phosphorylméthyl)-phthalate de diméthyle et 9,5 g (44,7 mmol) de 5-(2-éthyl-[1,3]dioxolan-2-yl)-thiophene-2-carbaldehyde (décrit à l'exemple 6(b)) sont dissous dans 200 ml de THF anhydre. 6,02 g (53,7 mmol) de *tert*-butylate de potassium sont ajoutés, et le mélange est agité pendant 24 h. Après traitement usuel et chromatographie sur gel de silice (éluant heptane 8 - acétate d'éthyle 2), le produit désiré est obtenu sous forme d'huile jaune (m = 9,53 g, R = 53%).
b) 4-{2-[5-(2-Ethyl-[1,3]dioxolan-2-yl)-thiophen-2-yl]-éthyl}-phthalate de diméthyle
   9,3 g (23 mmol) de 4-{(E)-2-[5-(2-éthyl-[1,3]dioxolan-2-yl)-thiophen-2-yl]-vinyl}-phthalate de diméthyle sont dissous dans 150 ml de dioxanne, puis 1 ml de triéthylamine est ajouté. Le mélange est dégazé à l'aide d'un flux d'azote, puis 9,3 g de palladium sur charbon 5% sont additionnés au milieu réactionnel. Une pression positive d'hydrogène est maintenue dans le milieu réactionnel pendant 5 heures, avec chauffage à 80°C. Après refroidissement, puis filtration sur Célite et évaporation, le produit désiré est obtenu sous forme d'huile jaune (m = 9,4 g ; R = 100%).
c) (4-{2-[5-(2-Ethyl-[1,3]dioxolan-2-yl)-thiophen-2-yl]-éthyl}-2-hydroxyméthyl-phényl)-méthanol
   De manière analogue à l'exemple 6(f) par réaction de 9,3 g (23 mmol) de 4-{2-[5-(2-éthyl-[1,3]dioxolan-2-yl)-thiophen-2-yl]-éthyl}-phthalate de diméthyle avec 2,2 g (58 mmol) d'hydrure double de lithium et d'aluminium. Le produit désiré est obtenu sous forme d'huile incolore (m = 8,7 g ; R = 100%).
d) 1-{5-[2-(3,4-Bis-hydroxyméthyl-phényl)-éthyl]-thiophen-2-yl}-propan-1-one
   De manière analogue à l'exemple 6(g) par réaction de 8,7 g (23 mmol) de (4-{2-[5-(2-éthyl-[1,3]dioxolan-2-yl)-thiophen-2-yl]-éthyl}-2-hydroxyméthyl-phényl)-méthanol avec une solution d'acide paratoluènesulfonique dans un mélange acétone/eau. Le produit désiré est obtenu sous forme de poudre blanche (m = 5,41 g ; R = 71 %).
e) 1-{5-[2-(3,4-Bis-(*tert*-butyl-diméthyl-silanyloxyméthyl)-phényl)-éthyl]-thiophen-2-yl}-propan-1-one
   De manière analogue à l'exemple 6(h), par réaction de 5,4 g ( 17,7 mmol) de 1-{5-[2-(3,4-bis-hydroxyméthyl-phényl)-éthyl]-thiophen-2-yl}-propan-1-one avec 3,37 g (49 mmol) d'imidazole et 5,86 g (39 mmol) de *tert*-butyldiméthylchlorosilane. Le produit désiré est obtenu sous forme d'huile incolore (m = 9,4 g ; R = 100% ).
f) Acide (E)-5-{5-[2-(3,4-bis-(*tert*-butyl-diméthyl-silanyloxyméthyl)-phényl)-éthyl]-thiophen-2-yl}-hept-4-enoique
   De manière analogue à l'exemple 6(i), par réaction de 5,08 g (9,5 mmol) de 1-{5-[2-(3,4-bis-(*tert*-butyl-diméthyl-silanyloxyméthyl)-phényl)-éthyl]-thiophen-2-yl}-propan-1-one avec 6,1 g (14 mmol) de bromure de (3-carboxypropyl)-triphénylphosphonium et 3,1 g (28 mmol) de *tert*-butylate de potassium. Le produit désiré est obtenu sous forme d'huile jaune (m = 2,7 g ; R = 48%).
g) (E)-5-{5-[2-(3,4-Bis-(*tert*-butyl-diméthyl-silanyloxyméthyl)-phényl)-éthyl]-thiophen-2-yl}-hept-4-enoate de méthyle
   De manière analogue à l'exemple 6(j), par réaction de 2,7 g (4,5 mmol) d'acide (E)-5-{5-[2-(3,4-bis-(*tert*-butyl-diméthyl-silanyloxyméthyl)-phényl)-éthyl]-thiophen-2-yl}-hept-4-enoique avec 680 mg (4,9 mmol) de carbonate de potassium et 1,38 ml (22,6 mmol) d'iodure de méthyle. Le produit désiré est obtenu sous forme d'huile jaune (m = 1,79 g ; R = 64%).
h) (E)-7-{5-[2-(3,4-Bis-(*tert*-butyl-diméthyl-silanyloxyméthyl)-phényl)-éthyl]-thiophen-2-yl}-3-éthyl-non-6-en-3-ol
   De manière analogue à l'exemple 6(k), par réaction de 1,79 g (2,9 mmol) de (E)-5-{5-[2-(3,4-bis-(*tert*-butyl-diméthyl-silanyloxyméthyl)-phényl)-éthyl]-thiophen-2-yl}-hept-4-enoate de méthyle avec 2,9 ml (8,7 mmol) de bromure d'éthylmagnesium 3,0 M. Le produit désiré est obtenu sous forme d'huile incolore (m = 710 mg ; R = 38%).
i) (E)-7-{5-[2-(3,4-Bis-hydroxyméthyl-phényl)-éthyl]-thiophen-2-yl}-3-éthyl-non-6-en-3-ol
   De manière analogue à l'exemple 6(1), par réaction de 710 mg (1,1 mmol) de (E)-7-{5-[2-(3,4-bis-(*tert*-butyl-diméthyl-silanyloxyméthyl)-phényl)-éthyl]-thiophen-2-yl}-3-éthyl-non-6-en-3-ol avec 3,3 ml (3,3 mmol) d'une solution de fluorure de tetrabutylammonium 1,0 M. Le produit désiré est obtenu sous forme de poudre blanche (PF 77°C ; m = 380 mg ; R = 83%).
   **RMN** ^{**1**}**H** (DMSO): 0,74 (t, 6H, J = 7,5 Hz); 0,97 (t, 3H, J = 7,4 Hz); 1,26-1,35 (m, 6H); 1,99-2,09 (m, 2H); 2,34 (q, 2H, J = 7,4 Hz); 2,80-2,86 (m, 2H); 2,92-2,98 (m, 2H); 3,86 (s, 1H); 4,43-4,47 (m, 4H); 4,95 (t, 1H, J = 4,7 Hz); 5,00 (t, 1H, J = 4,7 Hz); 5,63 (t, 1H, J = 7,3 Hz); 6,65 (d, 1H, J = 3,4 Hz); 6,75 (d, 1H, J = 3,4 Hz); 7,04 (d, 1H, J = 7,65 Hz); 7,20-7,22 (m, 2H).

### EXEMPLE 9

### (E)-7-[4-(3,4-Bis-hydroxyméthyl-phénoxyméthyl)-thiophen-2-yl]-3-éthyl-non-6-en-3-ol

a) 1-(4-Bromo-thiophen-2-yl)-propan-1-ol
   30 g (157 mmol) de 4-bromo-2-thiophene carboxaldehyde sont dissous dans 200 ml de THF anhydre. 104 ml (312 mmol) de bromure d'éthylmagnesium 3,0 M sont ajoutés lentement, et le milieu est agité 2 heures à température ambiante. Après traitement usuel, une huile orangée est obtenue (m = 33,5 g ; R = 96%).
b) 1-(4-Bromo-thiophen-2-yl)-propan-1-one
   33,5 g (151 mmol) de 1-(4-bromo-thiophen-2-yl)-propan-1-ol sont dissous dans 400 ml de dichlorométhane. 130 g (1,5 mol) de dioxyde de manganèse sont alors additionnés par fractions, et le milieu est agité à température ambiante pendant 24 heures. Après filtration sur Célite, puis concentration et chromatographie sur gel de silice (éluant heptane 9 - acétate d'éthyle 1), le produit désiré est obtenu sous forme de cristaux blancs (m = 26,6 g ; R = 80%).
c) Acide (E)-5-(4-Bromo-thiophen-2-yl)-hept-4-enoique
   De manière analogue à l'exemple 6(i), par réaction de 16 g (73 mmol) de 1-(4-bromo-thiophen-2-yl)-propan-1-one avec 47 g (109 mmol) de bromure de (3-carboxypropyl)-triphénylphosphonium et 24,6 g (219 mmol) de *tert*-butylate de potassium. Le produit désiré est obtenu sous forme de solide orangé (m = 16,7 g ; R = 80%).
d) (E)-5-(4-Bromo-thiophen-2-yl)-hept-4-enoate d'éthyle
   16,6 g (57 mmol) d'acide (E)-5-(4-bromo-thiophen-2-yl)-hept-4-enoique sont dissous dans 150 ml d'éthanol absolu, et 1 ml d'acide sulfurique concentré est ajouté. Le mélange est porté à reflux pendant 2 heures, puis refroidi. Après un traitement usuel et chromatographie sur gel de silice, le produit désiré est obtenu sous forme d'huile jaune (m = 14,1 g ; R = 77%).
e) (E)-7-(4-Bromo-thiophen-2-yl)-3-éthyl-non-6-en-3-ol
   De manière analogue à l'exemple 6(k), par réaction de 14,1 g (44,4 mmol) de (E)-5-(4-bromo-thiophen-2-yl)-hept-4-enoate d'éthyle avec 60 ml (180 mmol) de bromure d'éthylmagnesium 3,0 M. Le produit désiré est obtenu sous forme d'huile jaune (m = 12,8 mg ; R = 87%).
f) [(E)-5-(4-Bromo-thiophen-2-yl)-1,1-diéthyl-hept-4-enyloxy]-triéthyl-silane
   11,8 g (35,6 mmol) de (E)-7-(4-bromo-thiophen-2-yl)-3-éthyl-non-6-en-3-ol sont dissous dans 150 ml de dichlorométhane. 130 mg (1,1 mmol) de diméthylaminopyridine et 14,9 ml (107 mmol) de triéthylamine sont ajoutés. Le milieu réactionnel est refroidi à 0°C, et 12,1 ml (53,4 mmol) de triéthylsilyltrifluorométhanesulfonate sont additionnés goutte à goutte. Le milieu est ramené à température ambiante et agité 15 minutes puis versé dans 150 ml d'eau et extrait avec du dichlorométhane. Après chromatographie sur gel de silice (éluant heptane), le produit désiré est obtenu sous forme d'huile incolore (m = 15 g ; R = 100% ).
g) 5-((E)-1,5-Diéthyl-5-triéthylsilanyloxy-hept-1-enyl)-thiophene-3-carbaldehyde
   11 g (24,7 mmol) de [(E)-5-(4-bromo-thiophen-2-yl)-1,1-diéthyl-hept-4-enyloxy]-triéthyl-silane sont dissous dans 100 ml de THF anhydre et le mélange est refroidi à -78°C. 10,9 ml (27 mmol) de butyllithium 2,5 M sont additionnés lentement, et le mélange agité 15 minutes. Alors 2,1 ml (27 mmol) de diméthylformamide sont additionnés, et le milieu est agité pendant 30 minutes puis versé dans une solution aqueuse de chlorure d'ammonium. Après extraction avec de l'acétate d'éthyle et chromatographie sur gel de silice ( éluant heptane 9 - acétate d'éthyle 1), le produit est obtenu sous forme d'huile incolore (m = 4 g ; R = 41%).
h) [5-((E)-1,5-Diéthyl-5-triéthylsilanyloxy-hept-1-enyl)-thiophen-3-yl]-méthanol
   4 g (9,9 mmol) de 5-((E)-1,5-diéthyl-5-triéthylsilanyloxy-hept-1-enyl)-thiophene-3-carbaldehyde sont dissous dans 50 ml de THF et 50 ml de méthanol. 1 g (26 mmol) de borohydrure de sodium sont ajoutés par portions. Le milieu réactionnel est agité pendant 1 heure, puis versé dans 100 ml d'eau. Après purification par chromatographie sur gel de silice, le produit désiré est obtenu sous forme d'huile incolore (m = 4 g ; R = 100%).
i) 4-[5-((E)-1,5-Diéthyl-5-triéthylsilanyloxy-hept-1-enyl)-thiophen-3-ylméthoxy]-phthalate de diméthyle
   3,9 g (9,8 mmol) de [5-((E)-1,5-diéthyl-5-triéthylsilanyloxy-hept-1-enyl)-thiophen-3-yl]-méthanol sont dissous dans 100 ml de dichlorométhane et le mélange est refroidi à 0°C. 2 ml (15 mmol) de triéthylamine sont additionnés, suivis de 840 µL (10,7 mmol) de chlorure de méthanesulfonyle. Après 30 minutes d'agitation, le milieu est traité avec une solution de chlorure d'ammonium. Le résidu brut obtenu est ensuite dissous dans 100 ml de 2-butanone. A cette solution sont ajoutés: 100 mg (0,7 mmol) d'iodure de sodium, 1,6 g (11,6 mmol) de carbonate de potassium et 2 g (10 mmol) de 4-hydroxyphthalate de diméthyle. Le milieu réactionnel est porté à reflux pendant 15 heures, puis refroidi et filtré sur Célite. Le résidu obtenu est purifié par chromatographie sur colonne de silice (éluant Heptane 93 - acétate d'éthyle 7). Le produit désiré est obtenu sous forme d'huile incolore (m = 1,7 g ; R = 30%).
j) 4-[5-((E)-1,5-Diéthyl-5-hydroxy-hept-1-enyl)-thiophen-3-ylméthoxy]-phthalate de diméthyle
   1,7 g (2,9 mmol) de 4-[5-((E)-1,5-diéthyl-5-triéthylsilanyloxy-hept-1-enyl)-thiophen-3-ylméthoxy]-phthalate de diméthyle sont dissous dans 50 ml de THF anhydre. 3,5 ml (3,5 mmol) de fluorure de tetrabutylammonium (1,0 M dans le THF) sont additionnés et le milieu est agité à 60°C pendant 3 heures. Après traitement usuel et purification sur gel de silice (éluant heptane 70 - acétate d'éthyle 30), le produit désiré est obtenu sous forme d'huile jaune (m = 710 mg ; R = 51%).
k) (E)-7-[4-(3,4-Bis-hydroxyméthyl-phénoxyméthyl)-thiophen-2-yl]-3-éthyl-non-6-en-3-ol
   480 mg (1,1 mmol) de 4-[5-((E)-1,5-diéthyl-5-hydroxy-hept-1-enyl)-thiophen-3-ylméthoxy]-phthalate de diméthyle sont dissous dans 10 ml d'éther éthylique. Cette solution est ajoutée à une suspension de 130 mg (3,4 mmol) d'hydrure double de lithium et d'aluminium, et le milieu réactionnel est agité pendant 15 minutes. Le milieu réactionnel est alors traité par addition successive de 130 µL d'eau, 130 µL de soude 15% et 400 µL d'eau. Après filtration et chromatographie sur gel de silice (éluant heptane 2 - acétate d'éthyle 8), le produit désiré est obtenu sous forme de cristaux blancs (PF 63-64°C ; m = 380 mg ; R = 90%).
   **RMN** ^{**1**}**H** (DMSO): 0,63 (t, 6H, J = 7,5 Hz); 0,87 (t, 3H, J = 7,4 Hz); 1,16-1,24 (m, 6H); 1,90-2,00 (m, 2H); 2,26 (q, 2H, J = 7,4 Hz); 3,76 (s, 1H); 4,27 (d, 2H, J = 5,3 Hz); 4,36 (d, 2H, J = 5,3 Hz); 4,77 (t, 1H, J = 5,3 Hz); 4,94 (t, 1H, J = 5,3 Hz); 5,03 (s, 2H); 5,62 (t, 1H, J = 7,3 Hz); 6,67-6,76 (m, 2H); 6,88 (d, 2H, J = 2,5 Hz); 7,12 (d, 1H, J = 13,8 Hz).

### EXEMPLE 10

### (E)-7-{4-[2-(3,4-Bis-hydroxyméthyl-phényl)-éthyl]-thiophen-2-yl}-3-éthyl-non-6-en-3-ol

a) 2-(4-Bromo-thiophen-2-yl)-2-éthyl-[1,3]dioxolane
   10,5 g (48 mmol) de 1-(4-bromo-thiophen-2-yl)-propan-1-one (décrit à l'exemple 9(b)) sont dissous dans 150 ml de toluène. 13,4 ml (239 mmol) d'éthylène glycol et 450 mg (2,4 mmol) d'acide para-toluènesulfonique sont ajoutés. Le montage est équipé d'un appareil de distillation de type Dean-Stark, et le milieu réactionnel est chauffé à 130°C. Après 24 heures à reflux, le mélange est traité avec une solution de bicarbonate de sodium, et extraite avec de l'acétate d'éthyle. Le produit brut obtenu est le produit désiré (m = 12,2 g ; R = 100%).
b) 2-(2-Ethyl-[1,3]dioxolan-2-yl)-thiophene-4-carbaldehyde
   De manière analogue à l'exemple 9(g), par réaction de 12 g (45,6 mmol) de 2-(4-bromo-thiophen-2-yl)-2-éthyl-[1,3]dioxolane avec 20 ml (50 mmol) de butyllithium 2,5 M et 3,9 ml (50 mmol) de diméthylformamide. Le produit désiré est obtenu sous forme d'huile jaune ( m = 4,6 g, R = 48% ).
c) 4-{(E)-2-[2-(2-Ethyl-[1,3]dioxolan-2-yl)-thiophen-4-yl]-vinyl}-phthalate de diméthyle
   De manière analogue à l'exemple 8(a), par réaction de 4,6 g (21,6 mmol) de 2-(2-éthyl-[1,3]dioxolan-2-yl)-thiophene-4-carbaldehyde avec 8,9 g (26 mmol) de 4-(diéthoxyphosphorylméthyl)-phthalate de diméthyle et 2,9 g (26 mmol) de *tert*-butylate de potassium. Le produit désiré est obtenu sous forme d'huile jaune (m = 7 g ; R = 87%).
d) 4-{2-[2-(2-Ethyl-[1,3]dioxolan-2-yl)-thiophen-4-yl]-éthyl}-phthalate de diméthyle
   De manière analogue à l'exemple 8(b) par réaction de 7 g (19 mmol) de 4-{(E)-2-[2-(2-éthyl-[1,3]dioxolan-4-yl)-thiophen-3-yl]-vinyl}-phthalate de diméthyle avec 7 g de palladium sur charbon 5%, le produit désiré est obtenu sous forme d'huile incolore (m = 6,7 g ; R = 95%).
e) (4-{2-[2-(2-Ethyl-[1,3]dioxolan-2-yl)-thiophen-4-yl]-éthyl}-2-hydroxyméthyl-phényl)-méthanol
   De manière analogue à l'exemple 6(f) par réaction de 6,7 g (18 mmol) de 4-{2-[2-(2-éthyl-[1,3]dioxolan-2-yl)-thiophen-4-yl]-éthyl}-phthalate de diméthyle avec 1,6 g (43 mmol) d'hydrure double de lithium et d'aluminium. Le produit désiré est obtenu sous forme d'huile incolore (m = 5,3 g ; R = 85%).
f) 1-{4-[2-(3,4-Bis-hydroxyméthyl-phényl)-éthyl]-thiophen-2-yl}-propan-1-one
   De manière analogue à l'exemple 6(g) par réaction de 5,3 g (15 mmol) de (4-{2-[2-(2-éthyl-[1,3]dioxolan-2-yl)-thiophen-4-yl]-éthyl}-2-hydroxyméthyl-phényl)- méthanol avec une solution d'acide para-toluène sulfonique dans un mélange acétone - eau. Le produit désiré est obtenu sous forme de poudre blanche (m = 4 g ; R = 86 %).
g) 1-{4-[2-(3,4-Bis-(*tert*-butyl-diméthyl-silanyloxyméthyl)-phényl)-éthyl]-thiophen-2-yl}-propan-1-one
   De manière analogue à l'exemple 6(h), par réaction de 3,8 g ( 12 mmol) de 1-{4-[2-(3,4-bis-hydroxyméthyl-phényl)-éthyl]-thiophen-2-yl}-propan-1-one avec 2,1 g (31 mmol) d'imidazole et 4,1 g (27,5 mmol) de *tert*-butyldiméthylchlorosilane. Le produit désiré est obtenu sous forme d'huile incolore (m = 6,8 g ; R = 100%).
h) Acide (E)-5-{4-[2-(3,4-Bis-(*tert*-butyl-diméthyl-silanyloxyméthyl)-phényl)-éthyl]-thiophen-2-yl}-hept-4-enoique
   De manière analogue à l'exemple 6(i), par réaction de 3 g (5,6 mmol) de 1-{4-[2-(3,4-bis-(*tert*-butyl-diméthyl-silanyloxyméthyl)-phényl)-éthyl]-thiophen-2-yl}-propan-1-one avec 3,6 g (8,4 mmol) de bromure de (3-carboxypropyl)-triphénylphosphonium et 1,9 g (17 mmol) de *tert*-butylate de potassium. Le produit désiré est obtenu sous forme d'huile jaune (m = 1,7 g, R = 50%).
i) (E)-5-{4-[2-(3,4-Bis-(*tert*-butyl-diméthyl-silanyloxyméthyl)-phényl)-éthyl]-thiophen-2-yl}-hept-4-enoate de méthyle
   De manière analogue à l'exemple 6(j), par réaction de 1,6 g (2,6 mmol) d'acide (E)-5-{4-[2-(3,4-bis-(*tert*-butyl-diméthyl-silanyloxyméthyl)-phényl)-éthyl]-thiophen-2-yl}-hept-4-enoique avec 400 mg (2,9 mmol) de carbonate de potassium et 810 µL (13 mmol) d'iodure de méthyle. Le produit désiré est obtenu sous forme d'huile jaune (m = 1,4 g ; R = 86%).
j) (E)-7-{4-[2-(3,4-Bis-(*tert*-butyl-diméthyl-silanyloxyméthyl)-phényl)-éthyl]-thiophen-2-yl}-3-éthyl-non-6-en-3-ol
   De manière analogue à l'exemple 6(k), par réaction de 1,4 g (2,3 mmol) de (E)-5-{4-[2-(3,4-bis-(*tert*-butyl-diméthyl-silanyloxyméthyl)-phényl)-éthyl]-thiophen-2-yl}-hept-4-enoate de méthyle avec 3 ml (9 mmol) de bromure d'éthylmagnesium 3,0 M. Le produit désiré est obtenu sous forme d'huile incolore (m = 1 g, R = 69%).
k) (E)-7-{4-[2-(3,4-Bis-hydroxyméthyl-phényl)-éthyl]-thiophen-2-yl}-3-éthyl-non-6-en-3-ol
   De manière analogue à l'exemple 6(1), par réaction de 1 g (1,55 mmol) de (E)-7-{4-[2-(3,4-bis-(*tert*-butyl-diméthyl-silanyloxyméthyl)-phényl)-éthyl]-thiophen-2-yl}-3-éthyl-non-6-en-3-ol avec 3,7 ml (3,7mmol) d'une solution de fluorure de tetrabutylammonium 1,0 M. Le produit désiré est obtenu sous forme de cristaux blancs (PF 85-86°C ; m = 580 mg ; R = 90%).
   **RMN** ^{**1**}**H** (DMSO): 0.89 (t, 6H, J = 7,6 Hz); 1,12 (t, 3H, J = 7,4 Hz); 1,42-1,51 (m, 6H); 2,15-2,24 (m, 2H); 2,50 (q, 2H, J = 7,4 Hz); 2,96-3,01 (m, 2H); 3,08-3,14 (m, 2H); 4,00 (s, 1H); 4,58-4,62 (m, 4H); 5,11 (t, 1H, J = 4,7 Hz); 5,15 (t, 1H, J = 4,8 Hz); 5,79 (t, 1H, J = 7,4 Hz); 6,81 (d, 1H, J = 3,5 Hz); 6,90 (d, 1H, J = 3,5 Hz); 7,20 (d, 1H, J = 7,7 Hz); 7,35-7,38 (m, 2H).

### EXEMPLE 11

### (E)-7-[5-(3,4-Bis-hydroxyméthyl-phénoxyméthyl)-thiophen-3-yl]-3-éthyl-4,4-diméthyl-non-6-en-3-ol

a) (3-Bromo-thiophen-5-yl)-méthanol
   De manière analogue à l'exemple 9(h), par réaction de 20,5 g (107 mmol) de 3-bromothiophène-3-carboxaldehyde avec 4 g (110mmol) de borohydrure de sodium. Le produit désiré est obtenu sous forme d'huile jaune (m = 20 g ; R = 100%).
b) 3-(3-Bromo-thiophen-5-ylméthoxy)-phthalate de diméthyle
   De manière analogue à l'exemple 9(i), par réaction de 20g (103 mmol) de (3-bromo-thiophen-5-yl)-méthanol avec 21,6 ml (155 mmol) de triéthylamine et 8,8 ml (114 mmol) de chlorure de méthanesulfonyle, puis 500 mg (3,5 mmol) d'iodure de sodium, 14,3 g (103 mmol) de carbonate de potassium et 21,7 g (103 mmol) de 4-hydroxyphthalate de diméthyle. Le produit désiré est obtenu sous forme de cristaux orangés (PF 65°C ; m = 27 g ; R = 74%).
c) [5-(3-Bromo-thiophen-5-ylméthoxy)-2-hydroxyméthyl-phényl]-méthanol
   27 g (76 mmol) de 4-(3-bromo-thiophen-5-ylméthoxy)-phthalate de diméthyle sont dissous dans 200 ml de THF anhydre. Cette solution est additionnée goutte à goutte à une suspension de 4 g (183 mmol) de borohydrure de lithium dans 50 ml de THF. Le milieu réactionnel est porté à reflux pendant 24h, puis refroidi et versé sur 200 g de glace. Après extraction avec de l'éther puis chromatographie sur colonne de silice (éluant 5 heptane - 5 acétate d'éthyle), le produit désiré est obtenu sous forme d'huile jaune (m = 22,1 g ; R = 88%).
d) 5-[3,4-Bis-(*tert*-butyl-diméthyl-silanyloxyméthyl)-phénoxyméthyl]-3-bromo-thiophene
   De manière analogue à l'exemple 6(h), par réaction de 22 g ( 67 mmol) [5-(3-bromo-thiophen-5-ylméthoxy)-2-hydroxyméthyl-phényl]-méthanol avec 11,4 g (167 mmol) d'imidazole et 22 g (147 mmol) de *tert*-butyldiméthylchlorosilane. Le produit désiré est obtenu sous forme d'huile incolore (m = 34 g, R = 91%).
e) 5-[3,4-Bis-(*tert*-butyl-diméthyl-silanyloxyméthyl)-phénoxyméthyl]-thiophene-3-carbaldehyde
   De manière analogue à l'exemple 9(g), par réaction de 34 g (61 mmol) de 5-[3,4-bis-(*tert*-butyl-diméthyl-silanyloxyméthyl)-phénoxyméthyl]-3-bromo-thiophene avec 27 ml (67 mmol) de butyllithium 2,5 M et 5,2 ml (67 mmol) de diméthylformamide. Le produit brut obtenu (33,8 g ; R = 99%) est sous forme d'huile marron.
f) 1-{5-[3,4-Bis-(*tert*-butyl-diméthyl-silanyloxyméthyl)-phénoxyméthyl]-thiophen-3-yl}-propan-1-ol
   De manière analogue à l'exemple 9(a), par réaction de 33 g (65 mmol) de 5-[3,4-bis-(*tert*-butyl-diméthyl-silanyloxyméthyl)-phénoxyméthyl]-thiophene-3-carbaldehyde avec 44 ml (130 mmol) de bromure d'éthylmagnesium. Le produit est obtenu sous forme d'huile jaune (m = 28,4 g ; R = 88%).
g) 1-{5-[3,4-Bis-(*tert*-butyl-diméthyl-silanyloxyméthyl)-phénoxyméthyl]-thiophen-3-yl}-propan-1-one
   De manière analogue à l'exemple 9(b), par réaction de 28 g (52 mmol) de 1-{5-[3,4-bis-(*tert*-butyl-diméthyl-silanyloxyméthyl)-phénoxyméthyl]-thiophen-3-yl}-propan-1-ol avec 45 g (520 mmol) de dioxyde de manganese. Le produit désiré est obtenu sous forme d'huile orange (m = 26 g ; R = 93%).
h) Acide (E)-5-{5-[3,4-bis-(*tert*-butyl-diméthyl-silanyloxyméthyl)-phénoxyméthyl]-thiophen-3-yl}-hept-4-enoique
   De manière analogue à l'exemple 6(i), par réaction de 10 g (18,7 mmol) 1-{5-[3,4-bis-(*tert*-butyl-diméthyl-silanyloxyméthyl)-phénoxyméthyl]-thiophen-3-yl}-propan-1-one avec 12 g (28 mmol) de bromure de (3-carboxypropyl)-triphénylphosphonium et 6,3 g (56 mmol) de *tert*-butylate de potassium. Le produit désiré est obtenu sous forme d'huile brune (m = 5,4 g ; R = 48%).
i) (E)-5-{5-[3,4-Bis-(*tert*-butyl-diméthyl-silanyloxyméthyl)-phénoxyméthyl]-thiophen-3-yl}-hept-4-enoate de méthyle
   De manière analogue à l'exemple 6(j), par réaction de 5,4 g (8,9 mmol) d'acide (E)-5-{5-[3,4-bis-(*tert*-butyl-diméthyl-silanyloxyméthyl)-phénoxyméthyl]-thiophen-3-yl}-hept-4-enoique avec 1,3 g (9,4 mmol) de carbonate de potassium et 2,7 ml (44 mmol) d'iodure de méthyle. Le produit désiré est obtenu sous forme d'huile incolore (m = 3 g ; R = 54%).
j) (E)-5-{5-[3,4-Bis-(*tert*-butyl-diméthyl-silanyloxyméthyl)-phénoxyméthyl]-thiophen-3-yl}-2,2-diméthyl-hept-4-enoate de méthyle
   1,3 g (2,1 mmol) de (E)-5-{5-[3,4-bis-(*tert*-butyl-diméthyl-silanyloxyméthyl)-phénoxyméthyl]-thiophen-3-yl}-hept-4-enoate de méthyle sont dissous dans 20 ml de THF. Cette solution est additionnée à une solution de 3,2 mmol de diisopropylamidure de lithium dans 10 ml de THF, à -78°C. Après 10 minutes, 390 µL (6,2 mmol) d'iodure de méthyle sont additionnés, et le milieu réactionnel est ramené à température ambiante puis agité 12 h. Après le traitement usuel, le résidu brut obtenu est soumis une seconde fois aux mêmes conditions opératoires. Après chromatographie sur gel de silice (éluant heptane 97 - acétate d'éthyle 3), le produit désiré est obtenu sous forme d'huile incolore (m = 680 mg ; R = 50 %).
k) (E)-7-{5-[3,4-Bis-(*tert*-butyl-diméthyl-silanyloxyméthyl)-phénoxyméthyl]-thiophen-3-yl}-3-éthyl-4,4-diméthyl-non-6-en-3-ol
   De manière analogue à l'exemple 6(k), par réaction de 670 mg (1 mmol) de (E)-5-{5-[3,4-bis-(*tert*-butyl-diméthyl-silanyloxyméthyl)-phénoxyméthyl]-thiophen-3-yl}-2,2-diméthyl-hept-4-enoate de méthyle avec 1,4 ml (4,2 mmol) de bromure d'éthylmagnesium. Le produit désiré est obtenu sous forme d'huile incolore (m = 310 mg ; R = 44%).
l) (E)-7-[5-(3,4-Bis-hydroxyméthyl-phénoxyméthyl)-thiophen-3-yl]-3-éthyl-4,4-diméthyl-non-6-en-3-ol
   De Manière analogue à l'exemple 6(1), par réaction de 310 mg (0,46 mmol) de (E)-7-{5-[3,4-bis-(*tert*-butyl-diméthyl-silanyloxyméthyl)-phénoxyméthyl]-thiophen-3-yl}-3-éthyl-4,4-diméthyl-non-6-en-3-ol avec 1,1 ml (1,1 mmol) de bromure de tetrabutylammonium 1,0 M. Le produit désiré est obtenu sous forme d'huile incolore (m = 200 mg ; R = 98%).
   **RMN** ^{**1**}**H** (CDCl₃): 0.93-1,08 (m, 12H); 1,22 (t, 3H, J = 8,6 Hz); 1,62 (q, 4H, J = 8,5 Hz); 2,29 (d, 2H, J = 9,6 Hz); 2,48 (q, 2H, J = 8,6 Hz); 4,71 (s, 2H); 4,73 (s, 2H); 5,20 (s, 2H); 5,94 (t, 1H, J = 9,6 Hz); 6,90-6,95 (m, 1H); 7,04 (d, 1H, J = 3,9 Hz); 7.09 (d, 1H, J = 3,8 Hz); 7,22-7,31 (m, 2H).

### EXEMPLE 12

### (E)-7-[5-(3,4-Bis-hydroxyméthyl-phénoxyméthyl)-thiophen-3-yl]-3-éthyl-non-6-en-3-ol

a) (E)-7-{5-[3,4-Bis-(*tert*-butyl-diméthyl-silanyloxyméthyl)-phénoxyméthyl]-thiophen-3-yl}-3-éthyl-non-6-en-3-ol
   De manière analogue à l'exemple 6(k), par réaction de 1 g (1,6 mmol) de (E)-5-{5-[3,4-bis-(*tert*-butyl-diméthyl-silanyloxyméthyl)-phénoxyméthyl]-thiophen-3-yl}-hept-4-enoate de méthyle (décrit à l'exemple 11(i)) avec 2,1 ml (6,3 mmol) de bromure d'éthylmagnesium 3,0 M. Le produit désiré est obtenu sous forme d'huile incolore (m = 1 g ; R = 96%).
b) (E)-7-[5-(3,4-Bis-hydroxyméthyl-phénoxyméthyl)-thiophen-3-yl]-3-éthyl-non-6-en-3-ol
   De manière analogue à l'exemple 6(1), par réaction de 1 g (1,5 mmol) de (E)-7-{5-[3,4-bis-(*tert*-butyl-diméthyl-silanyloxyméthyl)-phénoxyméthyl]-thiophen-3-yl}-3-éthyl-non-6-en-3-ol avec 3,7 ml (3,7 mmol) de bromure de tetrabutylammonium 1,0 M. Le produit désiré est obtenu sous forme de cristaux blancs ( PF= 73-74°C ; m = 480 mg ; R = 74%).
   **RMN** ^{**1**}**H** (DMSO): 0,65 (t, 6H, J = 7,6 Hz); 0,84 (t, 3H, J = 7,5 Hz); 1,18-1,28 (m, 6H); 1,91-2,00 (m, 2H); 2,25 (q, 2H, J = 7,4 Hz); 3,76 (s, 1H); 4,28 (d, 2H; J = 4,9 Hz); 4,37 (d, 2H, J = 4,9 Hz); 4,79 (t, 1H, J = 5,0 Hz); 4,96 (t, 1H, J = 5,0 Hz); 5,06 (s, 2H); 5,67 (t, 1H, J = 7,3 Hz); 6,69-6,74 (m, 1H); 6,90 (d, 1H, J = 2,4 Hz); 7,10 (d, 1H, J = 8,3 Hz); 7,17-7,20 (m, 2H).

### EXEMPLE 13

### (3E.5E)-6-[5-(3,4-Bis-hydroxyméthyl-phénoxyméthyl)-thiophen-2-yl]-1.1.1-trifluoro-2-trifluorométhyl-octa-3,5-dien-2-ol

a) 2-[3,4-Bis-(*tert*-butyl-diméthyl-silanyloxyméthyl)-phénoxyméthyl]-5-((E)-4,4-dibromo-1-éthyl-buta-1,3-dienyl)-thiophene
   770 mg (11,7 mmol) de zinc, 3,09 g (11,7 mmol) de triphénylphosphine, 3,9 g (11,7 mmol) de tetrabromure de carbone sont agités pendant 30 minutes à température ambiante dans 150 ml de dichlorométhane. Une solution de 3,3 g (5,9 mmol) de (E)-3-{5-[3,4-bis-(*tert*-butyl-diméthyl-silanyloxyméthyl)-phénoxyméthyl]-thiophen-2-yl}-pent-2-enal (décrit à l'exemple 7(c)) dans 30 ml de dichlorométhane est alors additionnée goutte à goutte. Après 1h d'agitation à température ambiante, le milieu réactionnel est traité avec de l'eau et extrait au dichlorométhane. Après purification sur gel de silice (éluant heptane 95 - acétate d'éthyle 5), le produit désiré est obtenu sous forme d'huile brune (m = 3,9 g ; R = 93 %).
b) 2-[3,4-Bis-(*tert*-butyl-diméthyl-silanyloxyméthyl)-phénoxyméthyl]-5-((E)-1-éthyl-but-1-en-3-ynyl)-thiophene
   3,9 g (5,4 mmol) de 2-[3,4-bis-(*tert*-butyl-diméthyl-silanyloxyméthyl)-phénoxyméthyl]-5-((E)-4,4-dibromo-1-éthyl-buta-1,3-dienyl)-thiophene est dissous dans 100 ml de THF, et le mélange est refroidi à -78°C. 4,4 ml (11 mmol) de butyllithium 2,5 M sont ajoutés lentement, et le milieu réactionnel est agité à la même température pendant 1 heure. Après traitement usuel et chromatographie sur gel de silice (éluant heptane 96 - acétate d'éthyle 4), le produit désiré est obtenu sous forme d'huile brune.
c) (E)-6-{5-[3,4-Bis-(*tert*-butyl-diméthyl-silanyloxyméthyl)-phénoxyméthyl]-thiophen-2-yl}-1,1,1-trifluoro-2-trifluorométhyl-oct-5-en-3-yn-2-ol
   1,7 g (3 mmol) de 2-[3,4-bis-(*tert*-butyl-diméthyl-silanyloxyméthyl)-phénoxyméthyl]-5-((E)-1-éthyl-but-1-en-3-ynyl)-thiophene sont dissous dans 50 ml de THF, et le mélange est refroidi à -78°C. 1,3 ml (3,3 mmol) de butyllithium 2,5 M sont alors additionnés. Après 15 minutes d'agitation à la même température, un léger flux d'hexafluoroacétone (gaz) est introduit dans le milieu réactionnel. Après 20 minutes de réaction à -78°C, le flux de gaz est interrompu, et le milieu réactionnel traité de la manière usuelle. Le résidu obtenu est purifié par chromatographie sur gel de silice. Le produit désiré est obtenu sous forme d'huile jaune (1 g).
d) (3E,5E)-6-{5-[3,4-Bis-(*tert*-butyl-diméthyl-silanyloxyméthyl)-phénoxyméthyl]-thiophen-2-yl}-1,1,1-trifluoro-2-trifluorométhyl-octa-3,5-dien-2-ol
   1 g (1,3 mmol) de (E)-6-{5-[3,4-bis-(*tert*-butyl-diméthyl-silanyloxyméthyl)-phénoxyméthyl]-thiophen-2-yl}-1,1,1-trifluoro-2-trifluorométhyl-oct-5-en-3-yn-2-ol est dissous dans 10 ml de THF puis additionné à une suspension de 160 mg (4,2 mmol) d'hydrure double de lithium et d'aluminium 450 mg (8,4 mmol) de méthoxyde de sodium dans 20 ml de THF. Après 2 heures d'agitation à reflux, le milieu est refroidi, puis traité par 200 µL d'eau, 200 µL de soude 15% et 600 µL d'eau. Après filtration, le résidu est purifié par chromatographie sur gel de silice (éluant heptane 95 - acétate d'éthyle 5). Le produit désiré est obtenu sous forme d'huile jaune (210 mg).
e) (3E,5E)-6-[5-(3,4-Bis-hydroxyméthyl-phénoxyméthyl)-thiophen-2-yl]-1,1,1-trifluoro-2-trifluorométhyl-octa-3,5-dien-2-ol
   De manière analogue à l'exemple 6(1), par réaction de 200 mg de (3E,5E)-6-{5-[3,4-bis-(*tert*-butyl-diméthyl-silanyloxyméthyl)-phénoxyméthyl]-thiophen-2-yl}-1,1,1-trifluoro-2-trifluorométhyl-octa-3,5-dien-2-ol avec 700 µL (0,7 mmol) de bromure de tetrabutylammonium 1,0 M. Le produit désiré est obtenu sous forme de cristaux blancs ( PF= 113-114°C ; m = 70 mg).
   **RMN** ^{**1**}**H** (DMSO): 1,12 (t, 3H, J = 7,5 Hz); 2,71 (q, 2H, J = 7,5 Hz); 4.48 (d, 2H, J = 5,1 Hz); 4,57 (d, 2H, J = 5,1 Hz); 4,99 (t, 1H, J = 5,1 Hz); 5,15 (t, 1H, J = 5,1 Hz); 5,29 (s, 2H); 5,53 (d, 1H, J = 11,6 Hz); 6,89-7,04 (m, 2H); 7,10-7,35 (m, 5H); 8,50 (s, 1H).

### EXEMPLE 14

### (4E,6E)-7-[5-(3,4-Bis-hydroxymethyl-phenoxymethyl)-thiophen-3-yl]-3-ethyl-nona-4,6-dien-3-ol

a) (E)-3-{5-[3,4-Bis-(*tert*-butyl-diméthyl-silanyloxyméthyl)-phénoxyméthyl]-thiophen-3-yl}-pent-2-enoate d'éthyle
   De manière analogue à l'exemple 7(a) par réaction de 6 g (11,2 mmol) de 1-{5-[3,4-Bis-(*tert*-butyl-diméthyl-silanyloxyméthyl)-phénoxyméthyl]-thiophen-3-yl}-propan-1-one (préparé à l'exemple 11 (g)) avec 4,4 ml (22,4 mmol) de phosphonoacetate de triéthyle et 0,9 g (22,4 mmol) d'hydrure de sodium. Le produit désiré est séparé de son isomère Z et obtenu sous forme d'huile incolore (m = 5,3 g ; R = 78%).
b) (E)-3-{5-[3,4-Bis-(*tert*-butyl-diméthyl-silanyloxyméthyl)-phénoxyméthyl]-thiophen-3-yl}-pent-2-en-1-ol
   De manière analogue à l'exemple 7(b) par réaction de 2,3 g (3,8 mmol) de (E)-3-{5-[3,4-bis-(*tert*-butyl-diméthyl-silanyloxyméthyl)-phénoxyméthyl]-thiophen-3-yl}-pent-2-enoate d'éthyle avec 180 mg (4,7 mmol) d'hydrure double de lithium et d'aluminium. Le produit désiré (m = 2,1 g ; R = 100 %) est obtenu sous forme d'huile incolore.
c) (E)-3-{5-[3,4-Bis-(*tert*-butyl-diméthyl-silanyloxyméthyl)-phénoxyméthyl]-thiophen-3-yl}-pent-2-enal
   De manière analogue à l'exemple 7(c) par réaction de 2,1 g (3,7 mmol) de (E)-3-{5-[3,4-bis-(*tert*-butyl-diméthyl-silanyloxyméthyl)-phénoxyméthyl]-thiophen-3-yl}-pent-2-en-1-ol avec 4,9 g (56 mmol) de dioxyde de manganèse. Le produit brut ( 1,8 g) est l'aldéhyde attendu, obtenu avec un rendement de 86%.
d) (2E,4E)-5-{5-[3,4-Bis-(*tert*-butyl-diméthyl-silanyloxyméthyl)-phénoxyméthyl]-thiophen-3-yl}-hepta-2,4-dienoate d'éthyle
   De manière analogue à l'exemple 7(a), par réaction de 1,8 g (3,2 mmol) de (E)-3-{5-[3,4-bis-(*tert*-butyl-diméthyl-silanyloxyméthyl)-phénoxyméthyl]-thiophen-3-yl}-pent-2-enal avec 0,96 ml (4,8 mmol) de phosphonoacétate de triéthyle. Un isomère unique est obtenu: (2E,4E)-5-{5-[3,4-bis-(*tert*-butyl-diméthyl-silanyloxyméthyl)-phénoxyméthyl]-thiophen-3-yl}-hepta-2,4-dienoate d'éthyle est isolé sous forme d'huile jaune (m = 2 g ;R = 100%).
e) (4E,6E)-7-{5-[3,4-Bis-(*tert*-butyl-diméthyl-silanyloxyméthyl)-phénoxyméthyl]-thiophen-3-yl}-3-éthyl-nona-4,6-dien-3-ol
   De manière analogue à l'exemple 7(e) par réaction de 1,6 g (2,5 mmol) de (2E,4E)-5-{5-[3,4-bis-(*tert*-butyl-diméthyl-silanyloxyméthyl)-phénoxyméthyl]-thiophen-3-yl}-hepta-2,4-dienoate d'éthyle avec 17 mL d'une solution d'éthyl lithium (1,5 M). Le produit désiré est obtenu sous forme d'huile jaune (m = 160 mg, R = 10%).
f) (4E,6E)-7-[5-(3,4-Bis-hydroxyméthyl-phénoxyméthyl)-thiophen-3-yl]-3-éthyl-nona-4,6-dien-3-ol
   De manière analogue à l'exemple 7(f) par réaction de 155 mg (0,24 mmol) de (4E,6E)-7-{5-[3,4- bis-(*tert*-butyl-diméthyl-silanyloxyméthyl)-phénoxyméthyl]-thiophen-3-yl}-3-éthyl-nona-4,6-dien-3-ol avec 0,58 ml (0,58 mmol) d'une solution 1 M de fluorure de tetrabutylammonium. (4E,6E)-7-[5-(3,4-Bis-hydroxyméthyl-phénoxyméthyl)-thiophen-3-yl]-3-éthyl-nona-4,6-dien-3-ol est obtenu sous forme d'huile jaune (m = 80 mg ; R = 80% ).
   **RMN** ^{**1**}**H** (DMSO): 0,83 (t, J = 7,5 Hz, 6H); 1,08 (t, J = 7,6 Hz, 3H); 1,50 (q, J = 7,5 Hz, 4H); 2,54-2,60 (m, 2H); 4,33 (s, 1H); 4,48 (d, J = 5 Hz, 2H); 4,57 (d, J = 5 Hz, 2H); 4,98 (t, J = 5 Hz, 1H); 5,15 (t, J = 5 Hz, 1H); 5,27 (s, 2H); 5,80-5,84 (m, 1H); 6,55-6,59 (m, 2H); 6,91 (dd, J₁ = 2,6 Hz, J₂ = 8,3 Hz, 1H); 7,10 (d, J = 2,6 Hz, 1H); 7,29 (d, J = 8,2 Hz, 1H); 7,49 (s, 2H).

### EXEMPLE 15

### (4E,6E)-7-{4-[2-(3,4-Bis-hydroxymethyl-phenyl)-ethyl]-thiophen-2-yl}-3-ethyl-nona-4,6-dien-3-ol

a) (E)-3-(4-{2-[3,4-Bis-(*tert*-butyl-dimethyl-silanyloxymethyl)-phenyl]-ethyl}-thiophen-2-yl)-pent-2-enoate d'éthyle
   De manière analogue à l'exemple 7(a) par réaction de 3,6 g (6,7 mmol) de (E)-1-{4-[2-(3,4-Bis-(*tert*-butyl-diméthyl-silanyloxyméthyl)-phényl)-éthyl]-thiophen-2-yl}-propan-1-one (préparé à l'exemple 10(g)) avec 2,7 ml (13,5 mmol) de phosphonoacetate de triéthyle et 540 mg (13,5 mmol) d'hydrure de sodium. Le produit désiré est séparé de son isomère Z et obtenu sous forme d'huile incolore (m = 3,5 g ; R = 86%).
b) (E)-3-(4-{2-[3,4-Bis-(*tert*-butyl-dimethyl-silanyloxymethyl)-phenyl]-ethyl}-thiophen-2-yl)-pent-2-en-1-ol
   De manière analogue à l'exemple 7(b) par réaction de 2,3 g (3,8 mmol) de (E)-3-(4-{2-[3,4-bis-(*tert*-butyl-dimethyl-silanyloxymethyl)-phenyl]-ethyl}-thiophen-2-yl)-pent-2-enoate d'éthyle avec 180 mg (4,7 mmol) d'hydrure double de lithium et d'aluminium. Le produit désiré (m = 2,1 g ; R = 100 %) est obtenu sous forme d'huile incolore.
c) (E)-3-(4-{2-[3,4-Bis-(*tert*-butyl-dimethyl-silanyloxymethyl)-phenyl]-ethyl}-thiophen-2-yl)-pent-2-enal
   De manière analogue à l'exemple 7(c) par réaction de 2,0 g (3,6 mmol) de (E)-3-(4-{2-[3,4-bis-(*tert*-butyl-dimethyl-silanyloxymethyl)-phenyl]-ethyl}-thiophen-2-yl)-pent-2-en-1-ol avec 4,9 g (56 mmol) de dioxyde de manganèse. Le produit brut ( 2,0 g) est l'aldéhyde attendu, obtenu avec un rendement quantitatif.
d) (2E,4E)-5-(4-{2-[3,4-Bis-(*tert*-butyl-dimethyl-silanyloxymethyl)-phenyl]-ethyl}-thiophen-2-yl)-hepta-2,4-dienoate d'éthyle
   De manière analogue à l'exemple 7(d), par réaction de 2 g (3,6 mmol) de (E)-3-(4-{2-[3,4-bis-(*tert*-butyl-dimethyl-silanyloxymethyl)-phenyl]-ethyl}-thiophen-2-yl)-pent-2-enal avec 1,1 ml (5,4 mmol) de phosphonoacétate de triéthyle. Un isomère unique est obtenu: (2E,4E)-5-(4-{2-[3,4-bis-(*tert*-butyl-dimethyl-silanyloxymethyl)-phenyl]-ethyl}-thiophen-2-yl)-hepta-2,4-dienoate d'éthyle est isolé sous forme d'huile jaune (m = 1,9 g ;R = 85%).
e) (4E,6E)-7-(4-{2-[3,4-Bis-(*tert*-butyl-dimethyl-silanyloxymethyl)-phenyl]-ethyl}-thiophen-2-yl)-3-ethyl-nona-4,6-dien-3-ol
   De manière analogue à l'exemple 7(e) par réaction de 1,9 g (3 mmol) de (2E,4E)-5-(4-{2-[3,4-bis-(*tert*-butyl-dimethyl-silanyloxymethyl)-phenyl]-ethyl}-thiophen-2-yl)-hepta-2,4-dienoate d'éthyle avec 20 mL d'une solution d'éthyl lithium (1,5 M). Le produit désiré est obtenu sous forme d'huile jaune (m = 448 mg, R = 24%).
f) (4E,6E)-7-{4-[2-(3,4-Bis-hydroxymethyl-phenyl)-ethyl]-thiophen-2-yl}-3-ethyl-nona-4,6-dien-3-ol
   De manière analogue à l'exemple 7(f) par réaction de 440 mg (0,68 mmol) de (4E,6E)-7-(4-{2-[3,4-Bis-(*tert*-butyl-dimethyl-silanyloxymethyl)-phenyl]-ethyl}-thiophen-2-yl)-3-ethyl-nona-4,6-dien-3-ol avec 1,5 ml (1,5 mmol) d'une solution 1 M de fluorure de tetrabutylammonium. (4E,6E)-7-{4-[2-(3,4-Bis-hydroxymethyl-phenyl)-ethyl]-thiophen-2-yl}-3-ethyl-nona-4,6-dien-3-ol est obtenu sous forme d'huile jaune (m = 200 mg ; R = 71% ).
   **RMN** ^{**1**}**H** (DMSO): 0,57 (t, J = 7,5 Hz, 6H); 0,88 (t, J = 7,6 Hz, 3H); 1,24 (q, J = 7,5 Hz, 6H); 2,29-2,34 (m, 2H); 2,66-2,72 (m, 2H); 2,79-2,85 (m, 2H); 4,07 (s, 1H); 4,27-4,31 (m, 4H); 4,79 (t, J = 5 Hz, 1H); 4,83 (t, J = 5 Hz, 1H); 5,54 (d, J = 14,7 Hz, 1H); 6,13 (d, J = 11,1 Hz, 1H); 6,28 (dd, J1 = 11,1 Hz, J2 = 14,7 Hz, 1H); 6,54 (d, J = 3,6 Hz, 1H); 6,72 (d, J = 3,6 Hz, 1H); 6,88 (dd, J₁ = 1,4 Hz, J₂ = 6,3 Hz, 1H); 7,05-7,07 (m, 2H)..

### EXEMPLE 16 : EXEMPLES DE FORMULATION

### 1) VOIE ORALE

(a) On prépare la composition suivante sous la forme d'un comprimé de 0,2 g

| | |
|---|---|
| Composé de l'exemple 1 | 0,005 g |
| Amidon prégélatinisé | 0,065 g |
| Cellulose microcristalline | 0,075 g |
| Lactose | 0,050 g |
| Stéarate de magnésium | 0,005 g |

Pour le traitement de l'ichtyose, on administre à un individu adulte 1 à 3 comprimés par jour pendant 1 à 12 mois selon la gravité du cas traité.
(b) On prépare une suspension buvable, destinée à être conditionnée en ampoules de 5 ml

| | |
|---|---|
| Composé de l'exemple 2 | 0,050 mg |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 1 à 12 mois selon la gravité du cas traité.
(c) On prépare la formulation suivante destinée à être conditionnée en gélules :

| | |
|---|---|
| Composé de l'exemple 4 | 0,0001 mg |
| Amidon de maïs | 0,060 g |
| Lactose q.s.p. | 0,300 g |

Les gélules utilisées sont constituées de gélatine, d'oxyde de titane et d'un conservateur.
Dans le traitement du psoriasis, on administre à un individu adulte, 1 gélule par jour pendant 1 à 12 mois.
(d) On prépare la formulation suivante destinée à être conditionnée en gélules :

| | |
|---|---|
| Composé de l'exemple 5 | 0,02 mg |
| Cyclosporine | 0,050 g |
| Amidon de maïs | 0,060 g |
| Lactose q.s.p. | 0,300 g |

Les gélules utilisées sont constituées de gélatine, d'oxyde de titane et d'un conservateur.
Dans le traitement du psoriasis, on administre à un individu adulte, 1 gélule par jour pendant 1 à 12 mois.

### 2) VOIE TOPIQUE

(a) On prépare la crème Eau-dans l'Huile non ionique suivante :

| | |
|---|---|
| Composé de l'exemple 10 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 1 à 12 mois.
(b) On prépare un gel en réalisant la formulation suivante :

| | |
|---|---|
| Composé de l'exemple 15 | 0,001 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 12 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 8 | 1,000 g |
| Benzylidène camphre | 4,000 g |
| Triglycérides d'acides gras | 3.1,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.
(e) On prépare la crème Huile dans l'Eau non ionique suivante :

| | |
|---|---|
| Composé de l'exemple 7 | 0,500 g |
| Acide rétinoique | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours pour traitement d'attaque et indéfiniment pour entretien.
(f) On prépare un gel topique en procédant au mélange des ingrédients suivants :

| | |
|---|---|
| Composé de l'exemple 11 | 0,050 g |
| Ethanol | 43,000 g |
| α-tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau 9,300 g | |
| Propylène glycol qsp. | 100,000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 14 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 1 à 2 fois par jour pendant 3 mois sur un cuir chevelu ayant subi une chute de cheveu et indéfiniment pour traitement d'entretien.
(h) On prépare une crème anti-acnéique en procédant au mélange des ingrédients suivants :

| | |
|---|---|
| Composé de l'exemple 5 | 0,050 g |
| Acide rétinoïque | 0.010 g |
| Mélange de stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 4 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose inflammatoire pendant 30 jours.
(j) On prépare la crème de type huile dans l'eau suivante :

| | |
|---|---|
| Acide lactique | 5,000 g |
| Composé de l'exemple 1 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" ..... par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronologique.
(k) On prépare l'onguent anhydre suivant :

| | |
|---|---|
| Composé de l'exemple 1 | 5,000 g |
| Huile de vaseline | 50,00 g |
| butylhydrotoluène | 0,050 g |
| vaseline blanche | qs 100 g |

Cet onguent est appliqué 2 fois par jour sur une peau atteinte de dermatose squameuse pendant 30 jours.

### 3) VOIE INTRALESIONNELLE

(a) On prépare la composition suivante :

| | |
|---|---|
| Composé de l'exemple 2 | 0,002 g |
| Oléate d'éthyle | qs 10 g |

Dans le traitement du mélanome malin, on injecte la composition à un individu adulte à une fréquence de 1 à 7 fois par semaine pendant 1 à 12 mois.
(b) On prépare la composition suivante :

| | |
|---|---|
| Composé de l'exemple 1 | 0,050 g |
| Huile d'olive | qs 2 g |

Dans le traitement du carcinome basocellulaire, on injecte la composition à un individu adulte à une fréquence de 1 à 7 fois par semaine pendant 1 à 12 mois.
(c) On prépare la composition suivante :

| | |
|---|---|
| Composé de l'exemple 3 | 0,1 mg |
| Huile de sésame | qs 2 g |

Dans le traitement du carcinome spinocellulaire, on injecte la composition à un individu adulte à une fréquence de 1 à 7 fois par semaine pendant 1 à 12 mois.
(d) On prépare la composition suivante :

| | |
|---|---|
| Composé de l'exemple 4 | 0,001mg |
| Benzoate de méthyle | qs 10 g |

Dans le traitement du carcinome du colon, on injecte la composition à un individu adulte à une fréquence de 1 à 7 fois par semaine pendant 1 à 12 mois.

### 4) VOIE INTRAVEINEUSE

(a) On prépare l'émulsion lipidique injectable suivante :

| | |
|---|---|
| Composé de l'exemple 4 | 0,001 mg |
| Huile de soja | 10,000 g |
| Phospholipide d'oeuf | 1,200 g |
| Glycérine | 2,500 g |
| Eau pour injectable q.s.p. | 100,000 g |

Dans le traitement du psoriasis, on injecte la composition à un individu adulte à une fréquence de 1 à 7 fois par semaine pendant 1 à 12 mois.
(b) On prépare l'émulsion lipidique injectable suivante :

| | |
|---|---|
| Composé de l'exemple 3 | 0,010 g |
| Huile de coton | 10,000 g |
| Lécithine de soja | 0,750 g |
| Sorbitol | 5,000 g |
| DL,α-Tocophérol | 0,100 g |
| Eau pour injectable q.s.p. | 100,000 g |

Dans le traitement de l'ichtyose, on injecte la composition à un individu adulte à une fréquence de 1 à 7 fois par semaine pendant 1 à 12 mois.
(c) On prépare l'émulsion lipidique injectable suivante :

| | |
|---|---|
| Composé de l'exemple 2 | 0,001 g |
| Huile de soja | 15,000 g |
| Monoglycérides acétylés | 10,000 g |
| Pluronic F-1 08 | 1,000 g |
| Glycérol | 2,500 g |
| Eau pour injectable q.s.p. | 100,000 g |

Dans le traitement de la leucémie, on injecte la composition à un individu adulte à une fréquence de 1 à 7 fois par semaine pendant 1 à 12 mois.
(d) On prépare la composition micelle mixte suivante :

| | |
|---|---|
| Composé de l'exemple 2 | 0,001 g |
| Lécithine | 16,930 g |
| Acide glycocholique | 8,850 g |
| Eau pour injectable q.s.p. | 100,000 g |

Dans le traitement du mélanome malin, on injecte la composition à un individu adulte à une fréquence de 1 à 7 fois par semaine pendant 1 à 12 mois.
(e) On prépare la composition de cyclodextrine suivante :

| | |
|---|---|
| Composé de l'exemple 1 | 0,1mg |
| βcyclodextrine | 0,100 g |
| Eau pour injectable q.s.p. | 10,000 g |

Dans le traitement du rejet de greffe, on injecte la composition à un individu adulte à une fréquence de 1 à 7 fois par semaine pendant 1 à 12 mois.
(f) On prépare la composition de cyclodextrine suivante :

| | |
|---|---|
| Composé de l'exemple 4 | 0,010 g |
| 2-hydroxypropylβcyclodextrine | 0,100 g |
| Eau pour injectable q.s.p. | 10,000 g |

Dans le traitement du cancer du rein, on injecte la composition à un individu adulte à une fréquence de 1 à 7 fois par semaine pendant 1 à 12 mois.

### EXEMPLE 17 : Exemple de test d'évaluation de l'activité biologique des composés de l'invention

L'activité agoniste VDR a été testée sur la lignée cellulaire HeLa, par cotransfection d'un vecteur d'expression du récepteur VDR humain et du plasmide rapporteur p240Hase-CAT qui contient la région - 1399 à +76 du promoteur de la 24-hydroxylase de rat, clonée en amont de la phase codante du gène de la chloramphénicol-acétyl-transférase (CAT). 18 heures après cotransfection, le produit test est ajouté dans le milieu. Après 18 heures de traitement, le dosage de l'activité CAT des lysats cellulaires est effectuée par un test Elisa. Les résultats sont exprimés en pourcentage de l'effet normalement observé avec 10⁻⁷ M de calcitriol.

L'activité agoniste a été caractérisée dans ce système de cotransfection, par la détermination de la dose nécessaire pour atteindre 50 % de l'activité maximale du produit (AC50).

| Composé testé | AC 50 (nM) |
|---|---|
| **Exemple 1** | 192 |
| **Exemple 2** | 267 |
| **Exemple 4** | 294 |
| **Exemple 6** | 61 |
| **Exemple 7** | 17 |
| **Exemple 8** | 29 |
| **Exemple 10** | 23 |
| **Exemple 12** | 655 |
| **Exemple 13** | 78 |

## Revendications

1. Composés; **caractérisés par le fait qu'**ils répondent à la formule générale (I) suivante : 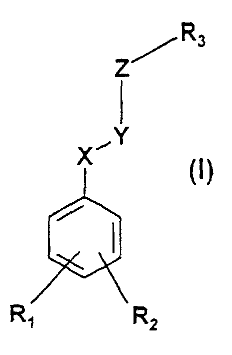 dans laquelle:
- **R**_{**1**} représente un atome d' hydrogène, un radical CH₃ ou un radical -(CH₂)₅-OR₄,
- **R**_{**2**} représente un radical -(CH₂)ₜ-OR₅,
s, t, R₄ et R₅ ayant les significations données ci-après,
- **X-Y** représente une liaison choisie parmi les liaisons de formules **(a)** à **(i)** suivantes: 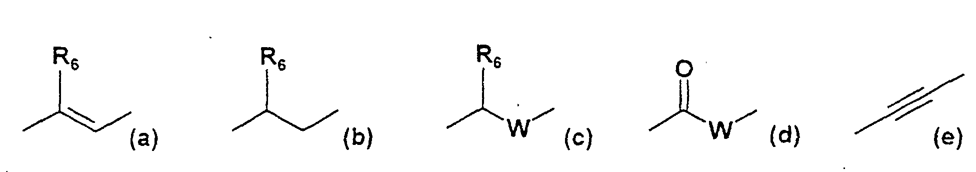 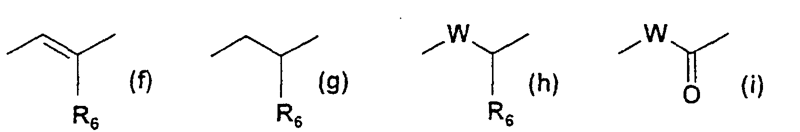 R₆ et W ayant les significations données ci-après,
- **Z** représente un cycle choisi parmi les cycles de formules **(j)** à **(n)** suivantes: 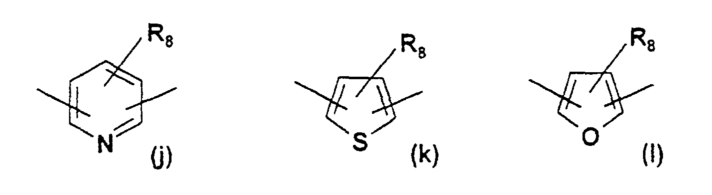 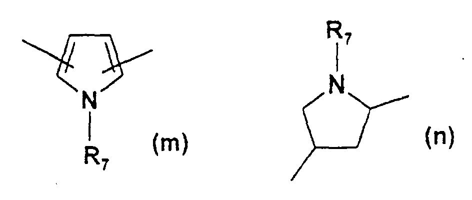 R₇ et R₈ ayant les significations données ci-après,
étant entendu que lorsque **Z** représente les cycles de formules **(k)**, **(l)**, ou **(m)**, alors **X-Y** ne peut pas représenter une liaison de formule **(c)**, **(d)**,
étant entendu que lorsque **Z** représente un cycle de formule **(n)**, alors **X-Y** représente de préférence une liaison de formule **(c)**, **(d)**,
- **R**_{**3**} représente une chaîne alkyle ayant de 4 à 8 atomes de carbone substituée par un ou plusieurs groupements hydroxyle, les groupements hydroxyles pouvant être protégés sous forme d'acétoxy, de méthoxy ou d'éthoxy, de triméthylsilyloxy, de tertiobutyldiméthylsilyloxy, de tétrahydropyranyloxy et éventuellement en outre :
- substituée par un ou plusieurs groupements alkyles inférieurs ou cycloalkyles et/ou
- substituée par un ou plusieurs atomes d'halogène et/ou
- substituée par un ou plusieurs groupements CF₃ et/ou
- dans laquelle un ou plusieurs atomes de carbone de la chaîne sont remplacés par des atomes d'oxygène, de soufre ou d'azote, les atomes d'azote pouvant être éventuellement substitués par des radicaux alkyles inférieurs et/ou
- dans laquelle une ou plusieurs liaisons simples de la chaîne sont remplacées par une ou plusieurs liaisons doubles et/ou triples,
- R3 étant positionné sur le cycle en para ou méta de la liaison X-Y,
- s et t identiques ou différents étant 1 ou 2,
- R₄ et R₅ identiques ou différents représentent un atome d'hydrogène, un radical acétyle, un radical benzoyle, un radical triméthylsilyle, un radical tertiobutyldiméthylsilyle, ou un radical tétrahydropyranyle,
- R₆ représente un atome d'hydrogène ou un radical alkyle inférieur,
- W représente un atome d'oxygène, de soufre ou un radical -NH- pouvant éventuellement être substitué par un radical alkyle inférieur,
- R₇ représente un atome d'hydrogène ou un radical alkyle inférieur,
- R₈ représente un atome d'hydrogène, un radical alkyle inférieur ou un atome d'halogène,
et les isomères optiques et géométriques desdits composés de formule (I) ainsi que leurs sels .

2. Composés selon la revendication 1, **caractérisés par le fait qu'**ils se présentent sous forme de sels d'un acide minéral ou organique, en particulier l'acide chlorhydrique, sulfurique, acétique, fumarique, hémisuccinique, maléique ou mandélique.

3. Composés selon l'une des revendications 1 ou 2, **caractérisés par le fait que** les radicaux alkyles inférieurs sont choisis parmi les radicaux méthyle, éthyle, isopropyle, tertiobutyle et hexyle.

4. Composés selon l'une des revendications précédentes, **caractérisés en ce que** le radical cycloalkyle correspond à un radical cyclopropyle, cyclopentyle ou cyclohexyle.

5. Composés selon l'une des revendications précédentes, **caractérisés en ce que** l'atome d'halogène correspond à un atome de fluor, de chlore ou de brome.

6. Composés selon la revendication 1, **caractérisés par le fait qu'**ils sont pris, seuls ou en mélanges, dans le groupe constitué par :
(E)-7-[5-(3,4-Bis-hydroxyméthyl-phenoxyméthyl)-thiophen-2-yl]-3-éthyl-oct-6-en-3-ol
(E)-7-[4-(3,4-Bis-hydroxyméthyl-phenoxyméthyl)-thiophen-2-yl]-3-éthyl-oct-6-en-3-ol
(E)-7-[2-(3,4-Bis-hydroxyméthyl-phenoxyméthyl)-thiophen-4-yl]-3-éthyl-oct-6-en-3-ol
(E)-7-[5-(3,4-Bis-hydroxyméthyl-phenoxyméthyl)-pyridin-3-yl]-3-éthyl-oct-6-en-3-ol
(E)-7-[6-(3,4-Bis-hydroxyméthyl-phenoxyméthyl)-pyridin-2-yl]-3-éthyl-non-6-en-3-ol
(E)-7-[5-(3,4-Bis-hydroxymethyl-phenoxymethyl)-thiophen-2-yl]-3-ethyl-non-6-en-3-ol
(4E,6E)-7-[5-(3,4-Bis-hydroxymethyl-phenoxymethyl)-thiophen-2-yl]-3-ethyl-nona-4,6-dien-3-ol
(3E,5E)-6-[5-(3,4-Bis-hydroxymethyl-phenoxymethyl)-thiophen-2-yl]-1,1,1-trifluoro-2-trifluoromethyl-octa-3,5-dien-2-ol
(4E,6E)-7-[5-(3,4-Bis-hydroxymethyl-phenoxymethyl)-thiophen-2-yl]-1,1,1,2,2-pentafluoro-3-pentafluoroethyl-nona-4,6-dien-3-ol
(E)-7-[5-(3,4-Bis-hydroxymethyl-phenoxymethyl)-thiophen-3-yl]-3-ethyl-4,4-dimethyl-non-6-en-3-ol
(E)-7-{5-[2-(3,4-Bis-hydroxymethyl-phenyl)-ethyl]-thiophen-2-yl}-3-ethyl-non-6-en-3-ol
(4E,6E)-7-{5-[2-(3,4-Bis-hydroxymethyl-phenyl)-ethyl]-thiophen-2-yl}-3-ethyl-nona-4,6-dien-3-ol
(3E,5E)-6-{5-[2-(3,4-Bis-hydroxymethyl-phenyl)-ethyl]-thiophen-2-yl}-1,1,1-trifluoro-2-trifluoromethyl-octa-3,5-dien-2-ol
((4E,6E)-7-{5-[2-(3,4-Bis-hydroxymethyl-phenyl)-ethyl]-thiophen-2-yl}-1,1,1,2,2-pentafluoro-3-pentafluoroethyl-nona-4,6-dien-3-ol
(4E,6E)-7-[5-(3,4-Bis-hydroxymethyl-benzylamino)-thiophen-2-yl]-3-ethyl-nona-4,6-dien-3-ol
(4E,6E)-7-{5-[(3,4-Bis-hydroxymethyl-benzyl)-methyl-amino]-thiophen-2-yl}-3-ethyl-nona-4,6-dien-3-ol
(4E,6E)-7-{5-[(3,4-Bis-hydroxymethyl-benzyl)-propyl-amino]-thiophen-2-yl}-3-ethyl-nona-4,6-dien-3-ol
(E)-7-[4-(3,4-Bis-hydroxymethyl-phenoxymethyl)-thiophen-2-yl]-3-ethyl-non-6-en-3-ol
(4E,6E)-7-[4-(3,4-Bis-hydroxymethyl-phenoxymethyl)-thiophen-2-yl]-3-ethyl-nona-4,6-dien-3-ol
(3E,5E)-6-[4-(3,4-Bis-hydroxymethyl-phenoxymethyl)-thiophen-2-yl]-1,1,1-trifluoro-2-trifluoromethyl-octa-3,5-dien-2-ol
(E)-7-{4-[2-(3,4-Bis-hydroxymethyl-phenyl)-ethyl]-thiophen-2-yl}-3-ethyl-non-6-en-3-ol
(4E,6E)-7-{4-[2-(3,4-Bis-hydroxymethyl-phenyl)-ethyl]-thiophen-2-yl}-3-ethyl-nona-4,6-dien-3-ol
(3E,5E)-6-{4-[2-(3,4-Bis-hydroxymethyl-phenyl)-ethyl]-thiophen-2-yl}-1,1,1-trifluoro-2-trifluoromethyl-octa-3,5-dien-2-ol
(E)-7-[5-(3,4-Bis-hydroxymethyl-phenoxymethyl)-thiophen-3-yl]-3-ethyl-non-6-en-3-ol
(4E,6E)-7-[5-(3,4-Bis-hydroxymethyl-phenoxymethyl)-thiophen-3-yl]-3-ethyl-nona-4,6-dien-3-ol
(3E,5E)-6-[5-(3,4-Bis-hydroxymethyl-phenoxymethyl)-thiophen-3-yl]-1,1,1-trifluoro-2-trifluoromethyl-octa-3,5-dien-2-ol
(E)-7- {5-[2-(3,4-Bis-hydroxymethyl-phenyl)-ethyl]-thiophen-3-yl}-3-ethyl-non-6-en-3-ol
(4E,6E)-7-{5-[2-(3,4-Bis-hydroxymethyl-phenyl)-ethyl]-thiophen-3-yl}-3-ethyl-nona-4,6-dien-3-ol
(3E,5E)-6-{5-[2-(3,4-Bis-hydroxymethyl-phenyl)-ethyl]-thiophen-3-yl}-1,1,1-trifluoro-2-trifluoromethyl-octa-3,5-dien-2-ol.

7. Composés selon la revendication 1, **caractérisés par le fait qu'**ils présentent l'une au moins des, et de préférence toutes les, caractéristiques suivantes :
- R₁ représente le radical -CH₃ ou -(CH₂)ₛOH,
- R₂ représente le radical -(CH₂)ₜOH,
- X-Y représente une liaison de formule (b), (c), (h) ou (g),
R₃ est choisi parmi
- une chaîne alkyle ou alkényle de 4 à 8 atomes de carbone substituée par au moins un radical hydroxyle et au moins un radical alkyle inférieur,
- ou une chaîne alkyle ou alkényle de 4 à 8 atomes de carbone substituée par au moins un radical hydroxyle, au moins un radical alkyle inférieur, et au moins un radical CF₃.

8. Utilisation d'un composé selon l'une quelconque des revendications précédentes pour la fabrication d'un médicament.

9. Utilisation d'un composé selon l'une quelconque des revendications 1 à 7 pour la fabrication d'un médicament destiné au traitement :
- des affections dermatologiques liées à un désordre de la kératinisation portant sur la différenciation et sur la prolifération telles que les acnés vulgaires, comédoniennes, polymorphes, rosacées, les acnés nodulokystiques, conglobata, les acnés séniles, les acnés secondaires telles que l'acné solaire, médicamenteuse ou professionnelle ;
- d'autres types de troubles de la kératinisation, telles que les ichtyoses, les états ichtyosiformes, la maladie de Darrier, les kératodermies palmoplantaires, les leucoplasies et les états leucoplasiformes, le lichen cutané ou muqueux (buccal) ;
- d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
- des proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que les lésions précancéreuses cutanées telles que les kératoacanthomes;
- des autres désordres dermatologiques tels que les dermatoses immunes telles le lupus érythémateux, les maladies immunes bulleuses ou les maladies du collagène, telle la sclérodermie;
- des affections dermatologiques ou générales à composante immunologique;
- des troubles de la fonction sébacée tels que l'hyperséborrhée de l'acné ou la séborrhée simple;
- des désordres cutanés dus à une exposition aux rayonnements U.V., du vieillissement de la peau, qu'il soit photo-induit ou chronologique, des pigmentations et les kératoses actiniques, ou toutes pathologies associées au vieillissement chronologique ou actinique;
- des troubles de la cicatrisation ou des vergetures,
- des affections inflammatoires telles que l'arthrite, des affections d'origine virale au niveau cutané ou général, telle que le syndrome de Kaposis;
- des troubles ophtalmologiques, notamment les cornéopathies;
- des états cancéreux ou précancéreux de cancers présentant ou pouvant être induits par des récepteurs de vitamine D, tels que le cancer du sein, la leucémie, les syndromes myélodysplasiques et les lymphomes, les carcinomes des cellules de l'épithélium malpighien et les cancers gastro-intestinaux, les mélanomes, et l'ostéosarcome;
- de l'alopécie de différentes origines, notamment l'alopécie due à la chimiothérapie ou aux rayonnements;
- des affections immunitaires, telles que les maladies auto-immunes, le diabète sucré de type 1, la sclérose en plaques, le lupus et les affections de type lupus, l'asthme, la glomérulonéphrite; des dysfonctionnements sélectifs du système immunitaire, telles que le SIDA, du rejet immun;
- des affections endocriniennes ;
- des affections **caractérisées par** une gestion anormale du calcium intracellulaire, des pathologies dans lesquelles le métabolisme calcique est impliqué, telle que l'ischémie musculaire;
- des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, ou les troubles de la fonction parathyroïdienne;
- des affections du système cardiovasculaire telles que l'artériosclérose ou l'hypertension ainsi que le diabète non-insulino dépendant,

10. Composition pharmaceutique, **caractérisée par le fait qu'**elle comprend, dans un support pharmaceutiquement acceptable, au moins l'un des composés tels que définis à l'une quelconque des revendications 1 à 7.

11. Composition selon la revendication 10, **caractérisée en ce que** la concentration en composé(s) selon l'une des revendications 1 à 7 est comprise entre 0,0001 % et 5 % en poids par rapport au poids total de la composition.

12. Composition cosmétique, **caractérisée par le fait qu'**elle comprend, dans un support cosmétiquement acceptable, au moins l'un des composés tels que définis à l'une quelconque des revendications 1 à 7.

13. Composition selon la revendication 12, **caractérisée en ce que** la concentration en composé(s) est comprise entre 0,001 % et 3 % en poids par rapport au poids total de la composition.

14. Utilisation d'une composition cosmétique telle que définie à l'une des revendications 12 ou 13 pour l'hygiène corporelle ou capillaire.

## Patentansprüche

1. Verbindungen, **dadurch gekennzeichnet, dass** sie der folgenden allgemeinen Formel (I) entsprechen: 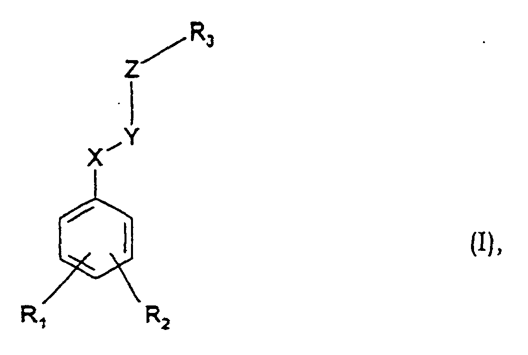 worin bedeuten:
- R₁ ein Wasserstoffatom, die Gruppe -CH₃ oder eine Gruppe -(CH₂)ₛOR₄;
- R₂ eine Gruppe -(CH₂)ₜOR₅, wobei s, t, R₄ und R₅ die nachstehend angegebenen Bedeutungen aufweisen ;
- X-Y eine Verbindungsgruppe, die unter den Verbindungsgruppen der folgenden Formeln (a) bis (i) ausgewählt ist: 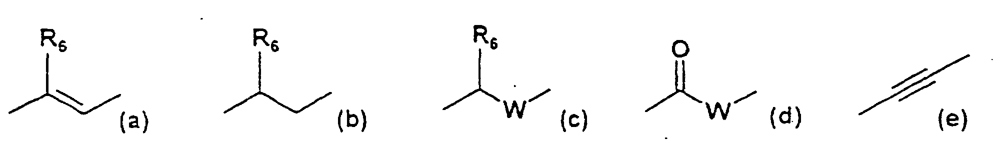 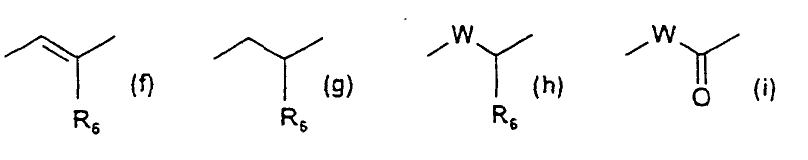
- Z einen Ring, der unter den Ringen der folgenden Formeln (j) bis (n) ausgewählt ist: 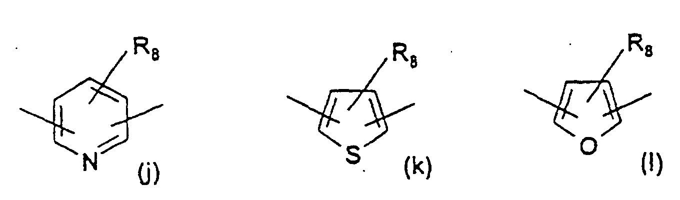 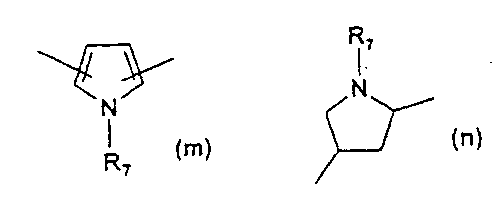 wobei R₇ und R₈ die nachstehend angegebenen Bedeutungen aufweisen;
mit der Maßgabe, dass X-Y keine Verbindungsgruppe der Formel (c) oder (d) bedeutet, wenn Z ein Ring der Formel (k), (l) oder (m) ist;
mit der Maßgabe, dass X-Y vorzugsweise eine Verbindungsgruppe der Formel (c) oder (d) bedeutet, wenn Z ein Ring der Formel (n) ist;
- R₃ eine Alkylgruppe mit 4 bis 8 Kohlenstoffatomen, die mit einer oder mehreren Hydroxygruppen substituiert ist, wobei die Hydroxygruppen in Form von Acetoxy, Methoxy, Ethoxy, Trimethylsilyloxy, *t*-Butyldimethylsilyloxy, Tetrahydropyranyloxy geschützt sein können, und gegebenenfalls ferner
- substituiert ist mit einer oder mehreren niederen Alkylgruppen oder Cycloalkylgruppen, und/oder
- substituiert ist mit einem oder mehreren Halogenatomen, und/oder
- substituiert ist mit einer oder mehreren CF₃-Gruppen, und/oder
- in dieser Gruppe ein oder mehrere Kohlenstoffatome der Kette durch Sauerstoff-, Schwefel- oder Stickstoffatome ersetzt sind, wobei die Stickstoffatome gegebenenfalls mit niederen Alkylgruppen substituiert sein können, und/oder
- in dieser Gruppe eine oder mehrere Einfachbindungen der Kette durch eine oder mehrere Doppelbindungen und/oder Dreifachbindungen ersetzt sind,
- wobei sich R₃ am Ring in para- oder meta-Stellung zur Verbindungsgruppe X-Y befindet,
- s und t, die gleich oder verschieden sind, 1 oder 2,
- R₄ und R₅, die gleich oder verschieden sind, Wasserstoff, Acetyl, Benzoyl, Trimethylsilyl, *t*-Butyldimethylsilyl oder Tetrahydropyranyl,
- R₆ ein Wasserstoffatom oder eine niedere Alkylgruppe,
- W ein Sauerstoffatom, ein Schwefelatom oder die Gruppe - NH-, die gegebenenfalls mit einer niederen Alkylgruppe substituiert sein kann,
- R₇ ein Wasserstoffatom oder eine niedere Alkylgruppe,
- R₈ ein Wasserstoffatom, eine niedere Alkylgruppe oder ein Halogenatom,
und die optischen und geometrischen Isomere der Verbindungen der Formel (I) sowie ihre Salze.

2. Verbindungen nach Anspruch 1, **dadurch gekennzeichnet, dass** sie in Form von Salzen mit einer anorganischen oder organischen Säure, insbesondere Salzsäure, Schwefelsäure, Essigsäure, Fumarsäure, Hemibernsteinsäure, Maleinsäure oder Mandelsäure, vorliegen.

3. Verbindungen nach Anspruch 1 oder 2, **dadurch gekennzeichnet, dass** die niederen Alkylgruppen unter Methyl, Ethyl, Isopropyl, *t*-Butyl und Hexyl ausgewählt sind.

4. Verbindungen nach einem der vorhergehenden Ansprüche, **dadurch gekennzeichnet, dass** die Cycloalkylgruppe Cyclopropyl, Cyclopentyl oder Cyclohexyl bedeutet.

5. Verbindungen nach einem der vorhergehenden Ansprüche, **dadurch gekennzeichnet, dass** das Halogenatom unter Fluor, Chlor oder Brom ausgewählt ist.

6. Verbindungen nach Anspruch 1, **dadurch gekennzeichnet, dass** sie unter den folgenden Verbindungen oder deren Gemischen ausgewählt sind:
- (E)-7-[5-(3,4-Bis-hydroxymethyl-phenoxymethyl)-thiophen-2-yl]-3-ethyl-oct-6-en-3-ol,
- (E)-7-[4-(3,4-Bis-hydroxymethyl-phenoxymethyl)-thiophen-2-yl]-3-ethyl-oct-6-en-3-ol,
- (E)-7-[2-(3,4-Bis-hydroxymethyl-phenoxymethyl)-thiophen-4-yl]-3-ethyl-oct-6-en-3-ol,
- (E)-7-[5-(3,4-Bis-hydroxymethyl-phenoxymethyl)-pyridin-3-yl]-3-ethyl-oct-6-en-3-ol,
- (E)-7-[6-(3,4-Bis-hydroxymethyl-phenoxymethyl)-pyridin-2-yl]-3-ethyl-non-6-en-3-ol,
- (E)-7-[5-(3,4-Bis-hydroxymethyl-phenoxymethyl)-thiophen-2-yl]-3-ethyl-non-6-en-3-ol,
- (4E,6E)-7-[5-(3,4-Bis-hydroxymethyl-phenoxymethyl)-thiophen-2-yl]-3-ethyl-nona-4,6-dien-3-ol,
- (3E,5E)-6-[5-(3,4-Bis-hydroxymethyl-phenoxymethyl)-thiophen-2-yl]-1,1, 1-trifluor-2-trifluormethyl-octa-3,5-dien-2-ol,
- (4E,6E)-7-[5-(3,4-Bis-hydroxymethyl-phenoxymethyl)-thiophen-2-yl]-1,1,1,2,2-pentafluor-3-pentafluorethyl-nona-4,6-dien-3-ol,
- (E)-7-[5-(3,4-Bis-hydroxymethyl-phenoxymethyl)-thiophen-3-yl]-3-ethyl-4,4-dimethyl-non-6-en-3-ol,
- (E)-7-{5-[2-(3,4-Bis-hydroxymethyl-phenyl)-ethyl]-thiophen-2-yl}-3-ethyl-non-6-en-3-ol,
- (4E,6E)-7-{5-[2-(3,4-Bis-hydroxymethyl-phenyl)-ethyl]-thiophen-2-yl}-3-ethyl-nona-4,6-dien-3-ol,
- (3E,5E)-6-{5-[2-(3,4-Bis-hydroxymethyl-phenyl)-ethyl]-thiophen-2-yl}-1,1,1-trifluor-2-trifluormethyl-octa-3,5-dien-2-ol,
- (4E,6E)-7-{5-[2-(3,4-Bis-hydroxymethyl-phenyl)-ethyl]-thiophen-2-yl}-1,1,1,2,2-pentafluor-3-pentafluorethyl-nona-4,6-dien-3-ol,
- (4E,6E)-7-[5-(3,4-Bis-hydroxymethyl-benzylamino)-thiophen-2-yl]-3-ethyl-nona-4,6-dien-3-ol,
- (4E,6E)-7-{5-[(3,4-Bis-hydroxymethyl-benzyl)-methyl-amino]-thiophen-2-yl}-3-ethyl-nona-4,6-dien-3-ol,
- (4E,6E)-7-{5-[(3,4-Bis-hydroxymethyl-benzyl)-propyl-amino]-thiophen-2-yl}-3-ethyl-nona-4,6-dien-3-ol,
- (E)-7-[4-(3,4-Bis-hydroxymethyl-phenoxymethyl)-thiophen-2-yl]-3-ethyl-non-6-en-3-ol,
- (4E,6E)-7-[4-(3,4-Bis-hydroxymethyl-phenoxymethyl)-thiophen-2-yl]-3-ethyl-nona-4,6-dien-3-ol,
- (3E,5E)-6-[4-(3,4-Bis-hydroxymethyl-phenoxymethyl)-thiophen-2-yl]-1,1,1-trifluor-2-trifluormethyl-octa-3,5-dien-2-ol,
- (E)-7-{4-[2-(3,4-Bis-hydroxymethyl-phenyl)-ethyl]-thiophen-2-yl}-3-ethyl-non-6-en-3-ol,
- (4E,6E)-7-{4-[2-(3,4-Bis-hydroxymethyl-phenyl)-ethyl]-thiophen-2-yl}-3-ethyl-nona-4,6-dien-3-ol,
- (3E,5E)-6-{4-[2-(3,4-Bis-hydroxymethyl-phenyl)-ethyl]-thiophen-2-yl}-1,1,1-trifluor-2-trifluormethyl-octa-3,5-dien-2-ol,
- (E)-7-[5-(3,4-Bis-hydroxymethyl-phenoxymethyl)-thiophen-3-yl]-3-ethyl-non-6-en-3-ol,
- (4E,6E)-7-[5-(3,4-Bis-hydroxymethyl-phenoxymethyl)-thiophen-3-yl]-3-ethyl-nona-4,6-dien-3-ol,
- (3E,5E)-6-[5-(3,4-Bis-hydroxymethyl-phenoxymethyl)-thiophen-3-yl]-1,1,1-trifluor-2-trifluormethyl-octa-3,5-dien-2-ol,
- (E)-7-{5-[2-(3,4-Bis-hydroxymethyl-phenyl)-ethyl]-thiophen-3-yl}-3-ethyl-non-6-en-3-ol,
- (4E,6E)-7-{5-[2-(3,4-Bis-hydroxymethyl-phenyl)-ethyl]-thiophen-3-yl}-3-ethyl-nona-4,6-dien-3-ol,
- (3E,5E)-6-{5-[2-(3,4-Bis-hydroxymethyl-phenyl)-ethyl]-thiophen-3-yl}-1,1,1-trifluor-2-trifluormethyl-octa-3,5-dien-2-ol.

7. Verbindungen nach Anspruch 1, **dadurch gekennzeichnet, dass** sie mindestens eine der folgenden Eigenschaften und vorzugsweise alle folgenden Eigenschaften aufweisen:
- R₁ bedeutet die Gruppe -CH₃ oder -(CH₂)ₛOH;
- R₂ bedeutet die Gruppe -(CH₂)ₜOH;
- X-Y bedeutet eine Verbindungsgruppe der Formel (b), (c), (h) oder (g);
- R₃ ist ausgewählt unter:
- einer Alkyl- oder Alkenylgruppe mit 4 bis 8 Kohlenstoffatomen, die mit mindestens einer Hydroxygruppe und mindestens einer niederen Alkylgruppe substituiert ist; oder
- einer Alkyl- oder Alkenylgruppe mit 4 bis 8 Kohlenstoffatomen, die mit mindestens einer Hydroxygruppe, mindestens einer niederen Alkylgruppe und mindestens einer CF₃-Gruppe substituiert ist.

8. Verwendung einer Verbindung nach einem der vorhergehenden Ansprüche für die Herstellung eines Arzneimittels.

9. Verwendung einer Verbindung nach einem der Ansprüche 1 bis 7 für die Herstellung eines Arzneimittels, das für die Behandlung der folgenden Erkrankungen vorgesehen ist:
- zur Behandlung von dermatologischen Erkrankungen, die mit einer Verhornungsstörung verbunden sind, die auf der Differenzierung und Proliferation beruht, beispielsweise zur Behandlung von Acne vulgaris, Acne comedonica, polymorpher Acne, Acne rosaceae, nodulocystischer Acne, Acne conglobata, Acne senilis, sekundären Akneformen, wie Acne solaris, Acne medikamentosa oder Acne professionalis;
- zur Behandlung von weiteren Arten von Verhomungsstörungen, wie Ichtyosis, ichtyosisartigen Zuständen, der Darier Krankheit, Palmoplantarkeratosen, Leucoplakien und leucoplakieformen Zuständen und Lichen der Haut oder der Schleimhäute (buccal);
- zur Behandlung weiterer dermatologischer Erkrankungen mit entzündlicher immunoallergischer Komponente mit oder ohne Proliferationsstörung der Zellen, insbesondere aller Arten von Psoriasis der Haut, der Schleimhäute oder der Nägel, Psoriasis arthropathica, Atopie der Haut wie Ekzemen, Atopie der Atemwege oder Hypertrophie des Zahnfleisches;
- zur Behandlung von Proliferationen der Dermis oder Epidermis, die gutartig oder bösartig und gegebenenfalls viralen Ursprungs sein können, wie Verrucae vulgares, Verrucae planae und Epidermodysplasia verruciformis, Papillomatosis oralis oder florida, T-Lymphomen und Proliferationen, die von UV-Strahlung induziert werden können, insbesondere im Falle von Epithelioma basocellulare und spinocellulare, sowie präkanzerösen Lesionen der Haut, wie Keratoakanthomen;
- zur Behandlung von weiteren dermatologischen Störungen, wie Immundermatosen, beispielsweise Lupus erythematodes, bullösen Immunerkrankungen und Kollagenerkrankungen, wie Sklerodermie;
- zur Behandlung von dermatologischen Störungen oder allgemeinen Störungen mit immunologischer Komponente;
- zur Behandlung von Funktionsstörungen der Talgdrüse, wie Hyperseborrhoe bei Akne oder einfache Seborrhoe;
- zur Behandlung von Hautstörungen, die von einer Exposition gegenüber UV-Strahlung herrühren, lichtinduzierter oder altersbedingter Hautalterung, aktinischen Pigmentierungen und Keratosen oder Pathologien, die mit der altersbedingten oder aktinischen Alterung zusammenhängen;
- zur Behandlung von Störungen der Wundheilung oder Streifen;
- zur Behandlung von entzündlichen Erkrankungen, wie Arthritis, Erkrankungen viraler Herkunft der Haut oder allgemeinen Erkrankungen viraler Herkunft, wie des Kaposi-Syndrom;
- zur Behandlung von ophthalmologischen Störungen, insbesondere Corneopathien;
- zur Behandlung von kanzerösen oder präkanzerösen Zuständen, die vorhanden sind oder von Vitamin D-Rezeptoren induziert werden können, wie Brustkrebs, Leukämie, myelodysplastischen Syndromen und Lymphomen, Stachelzellkarzinomen und Magen-Darm-Krebs, Melanomen und Osteosarkom;
- zur Behandlung von Alopezie unterschiedlichen Ursprungs, insbesondere durch Chemotherapie oder Strahlung verursachter Alopezie;
- zur Behandlung von Beeinträchtigungen der Immunfunktion, wie Autoimmunkrankheiten, Diabetes vom Typ 1, multiple Sklerose, Lupus und Erkrankungen vom Lupus-Typ, Asthma, Glomerulonephritis, selektive Funktionsstörungen des Immunsystems, wie AIDS, Immunrejektion;
- zur Behandlung von endokrinen Erkrankungen;
- zur Behandlung von Erkrankungen, die durch einen anormalen Haushalt des intrazellulären Calcium gekennzeichnet sind, Erkrankungen, bei denen der Calcium-Stoffwechsel beteiligt ist, wie Ischämie der Muskeln;
- zur Behandlung von Vitamin D-Mangel und weiteren Störungen der Homöostase von Mineralien im Plasma und in den Knochen, wie Rachitis, Osteomalazie, Osteoporose, insbesondere bei Frauen in den Wechseljahren, renale Osteodystrophie oder Störungen der Nebenschilddrüsenfunktion;
- zur Behandlung von Erkrankungen des cardiovasculären Systems, beispielsweise Arteriosklerose, Bluthochdruck sowie insulinunabhängiger Diabetes.

10. Pharmazeutische Zusammensetzung, **dadurch gekennzeichnet, dass** sie in einem pharmazeutisch akzeptablen Träger mindestens eine Verbindung nach einem der Ansprüche 1 bis 7 enthält.

11. Zusammensetzung nach Anspruch 10, **dadurch gekennzeichnet, dass** die Konzentration der Verbindung(en) nach einem der Ansprüche 1 bis 7 im Bereich von 0,0001 bis 5 Gew.-%, bezogen auf das Gesamtgewicht der Zusammensetzung, liegt.

12. Kosmetische Zusammensetzung, **dadurch gekennzeichnet, dass** sie in einem kosmetisch akzeptablen Träger mindestens eine Verbindung nach einem der Ansprüche 1 bis 7 enthält.

13. Zusammensetzung nach Anspruch 12, **dadurch gekennzeichnet, dass** die Konzentration der Verbindung(en) im Bereich von 0,001 bis 3 Gew.-%, bezogen auf das Gesamtgewicht der Zusammensetzung, liegt.

14. Verwendung einer kosmetischen Zusammensetzung nach einem der Ansprüche 12 oder 13 für die Körperpflege oder Haarpflege.

## Claims

1. Compounds, **characterized in that** they correspond to the general formula (I) below: 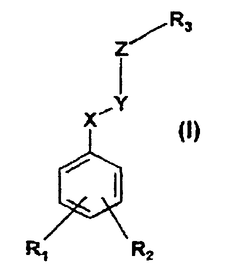 in which:
- **R**_{**1**} represents a hydrogen atom, a CH₃ radical or a radical -(CH₂)ₛ-OR₄,
- **R**_{**2**} represents a radical -(CH₂)ₜ-OR₅,
s, t, R₄ and R₅ having the meanings given below,
- **X-Y** represents a bonding group chosen from the bonding groups of formulae **(a)** to **(i)** below: 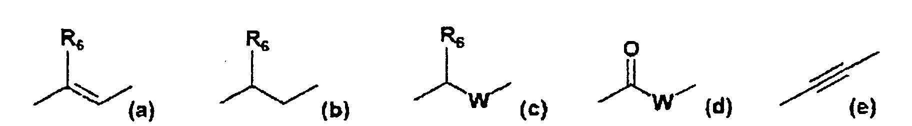 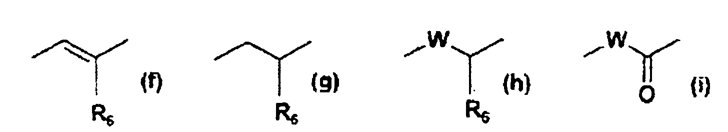 R₆ and W having the meanings given below,
- **Z** represents a ring chosen from the rings of formulae **(j)** to **(n)** below: 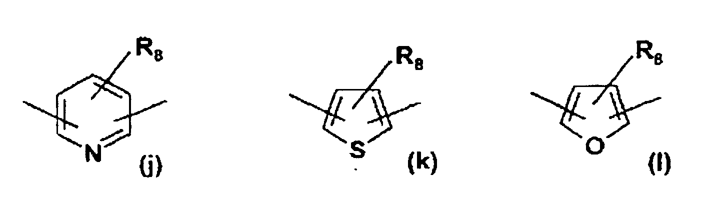 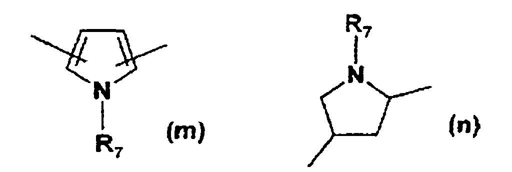 R₇ and R₈ having the meanings given below, it being understood that when **z** represents the rings of formula **(k), (l)** or **(m),** then **X-Y** cannot represent a bonding group of formula **(c)** or **(d),**
it being understood that when **Z** represents a ring of formula **(n),** then **X-Y** preferably represents a bonding group of formula **(c)** or **(d),**
- **R**_{**3**} represents an alkyl chain containing from 4 to 8 carbon atoms substituted with one or more hydroxyl groups, it being possible for the hydroxyl groups to be protected in the form of acetoxy, methoxy or ethoxy, trimethylsilyloxy, tert-butyldimethylsilyloxy, tetrahydropyranyloxy and optionally also:
- substituted with one or more lower alkyl or cycloalkyl groups and/or
- substituted with one or more halogen atoms and/or
- substituted with one or more CF₃ groups and/or
- in which one or more carbon atoms of the chain are replaced with oxygen, sulphur or nitrogen atoms, it being possible for the nitrogen atoms to be optionally substituted with lower alkyl radicals and/or
- in which one or more single bonds of the chain are replaced with one or more double and/or triple bonds,
- R3 being positioned on the ring para or meta to the bonding group X-Y,
- s and t, which may be identical or different, being 1 or 2,
- R₄ and R₅, which may be identical or different, represent a hydrogen atom, an acetyl radical, a benzoyl radical, a trimethylsilyl radical, a tert-butyldimethylsilyl radical or a tetrahydropyranyl radical,
- R₆ represents a hydrogen atom or a lower alkyl radical,
- W represents an oxygen or sulphur atom or an -NH- radical which can optionally be substituted with a lower alkyl radical,
- R₇ represents a hydrogen atom or a lower alkyl radical,
- R₈ represents a hydrogen atom, a lower alkyl radical or a halogen atom,
and the optical and geometrical isomers of the said compounds of formula (I), as well as the salts thereof.

2. Compounds according to Claim 1, **characterized in that** they are in the form of salts of an inorganic or organic acid, in particular hydrochloric acid, sulphuric acid, acetic acid, fumaric acid, hemisuccinic acid, maleic acid or mandelic acid.

3. Compounds according to either of Claims 1 and 2, **characterized in that** the lower alkyl radicals are chosen from methyl, ethyl, isopropyl, tert-butyl and hexyl radicals.

4. Compounds according to one of the preceding claims, **characterized in that** the cycloalkyl radical corresponds to a cyclopropyl, cyclopentyl or cyclohexyl radical.

5. Compounds according to one of the preceding claims, **characterized in that** the halogen atom corresponds to a fluorine, chlorine or bromine atom.

6. Compounds according to Claim 1, **characterized in that** they are taken, alone or as mixtures, from the group consisting of:
(E)-7-[5-(3,4-bis-hydroxymethyl-phenoxymethyl)-2-thienyl]-3-ethyloct-6-en-3-ol
(E)-7-[4-(3,4-bis-hydroxymethyl-phenoxymethyl)-2-thienyl]-3-ethyloct-6-en-3-ol
(E)-7-[2-(3,4-bis-hydroxymethyl-phenoxymethyl)-4-thienyl]-3-ethyloct-6-en-3-ol
(E)-7-[5-(3,4-bis-hydroxymethyl-phenoxymethyl)-3-pyridyl]-3-ethyloct-6-en-3-ol
(E)-7-[6-(3,4-bis-hydroxymethyl-phenoxymethyl)-2-pyridyl)-3-ethylnon-6-en-3-ol
(E)-7-[5-(3,4-bis-hydroxymethyl-phenoxymethyl)-2-thienyl]-3-ethylnon-6-en-3-ol
(4E,6E)-7-[5-(3,4-bis-hydroxymethyl-phenoxymethyl)-2-thienyl]-3-ethylnona-4,6-dien-3-ol
(3E,5E)-6-[5-(3,4-bis-hydroxymethyl-phenoxymethyl)-2-thienyl]-1,1,1-trifluoro-2-trifluoromethylocta-3,5-dien-2-ol
(4E,6E)-7-[5-(3,4-bis-hydroxymethyl-phenoxymethyl)-2-thienyl]-1,1,1,2,2-pentafluoro-3-pentafluoroethylnona-4,6-dien-3-ol
(E)-7-[5-(3,4-bis-hydroxymethyl-phenoxymethyl)-3-thienyl]-3-ethyl-4,4-dimethylnon-6-en-3-ol
(E)-7-{5-[2-(3,4-bis-hydroxymethyl-phenyl)ethyl]-2-thienyl}-3-ethylnon-6-en-3-ol
(4E,6E)-7-{5-[2-(3,4-bis-hydroxymethyl-phenyl)ethyl]-2-thienyl}-3-ethylnona-4,6-dien-3-ol
(3E,5E)-6-{5-[2-(3,4-bis-hydroxymethyl-phenyl)ethyl]-2-thienyl}-1,1,1-trifluoro-2-trifluoromethylocta-3,5-dien-2-ol
(4E,6E)-7-{5-[2-(3,4-bis-hydroxymethyl-phenyl)ethyl]-2-thienyl}-1,1,1,2,2-pentafluoro-3-pentafluoroethylnona-4,6-dien-3-ol
(4E,6E)-7-[5-(3,4-bis-hydroxymethyl-benzylamino)-2-thienyl]-3-ethylnona-4,6-dien-3-ol
(4E,6E)-7-{5-[(3,4-bis-hydroxymethyl-benzyl)methyl-amino]-2-thienyl}-3-ethylnona-4,6-dien-3-ol
(4E,6E)-7-{5-[(3,4-bis-hydroxymethyl-benzyl)propyl-amino]-2-thienyl}-3-ethylnona-4,6-dien-3-ol
(E)-7-[4-(3,4-bis-hydroxymethyl-phenoxymethyl)-2-thienyl]-3-ethylnon-6-en-3-ol
(4E,6E)-7-[4-(3,4-bis-hydroxymethyl-phenoxymethyl)-2-thienyl]-3-ethylnona-4,6-dien-3-ol
(3E,5E)-6-[4-(3,4-bis-hydroxymethyl-phenoxymethyl)-2-thienyl]-1,1,1-trifluoro-2-trifluoromethylocta-3,5-dien-2-ol
(E)-7-{4-[2-(3,4-bis-hydroxymethyl-phenyl)ethyl]-2-thienyl}-3-ethylnon-6-en-3-ol
(4E,6E)-7-{4-[2-(3,4-bis-hydroxymethyl-phenyl)ethyl]-2-thienyl}-3-ethylnona-4,6-dien-3-ol
(3E,5E)-6-{4-[2-(3,4-bis-hydroxymethyl-phenyl)ethyl]-2-thienyl}-1,1,1-trifluoro-2-trifluoromethylocta-3,5-dien-2-ol
(E)-7-[5-(3,4-bis-hydroxymethyl-phenoxymethyl)-3-thienyl]-3-ethylnon-6-en-3-ol
(4E,6E)-7-[5-(3,4-bis-hydroxymethyl-phenoxymethyl)-3-thienyl]-3-ethylnona-4,6-dien-3-ol
(3E,5E)-6-[5-(3,4-bis-hydroxymethyl-phenoxymethyl)-3-thienyl]-1,1,1-trifluoro-2-trifluoromethylocta-3,5-dien-2-ol
(E)-7-{5-[2-(3,4-bis-hydroxymethyl-phenyl)ethyl]-3-thienyl}-3-ethylnon-6-en-3-ol
(4E,6E)-7-{5-[2-(3,4-bis-hydroxymethyl-phenyl)ethyl]-3-thienyl}-3-ethylnona-4,6-dien-3-ol
(3E,5E)-6-{5-[2-(3,4-bis-hydroxymethyl-phenyl)ethyl]-3-thienyl}-1,1,1-trifluoro-2-trifluoromethylocta-3,5-dien-2-ol.

7. Compounds according to Claim 1, **characterized in that** they have at least one, and preferably all, of the following characteristics:
- R₁ represents a -CH₃ or -(CH₂)ₛOH radical,
- R₂ represents a radical -(CH₂)ₜOH,
- X-Y represents a bonding group of formula (b), (c), (h) or (g),
R₃ is chosen from
- an alkyl or alkenyl chain of 4 to 8 carbon atoms substituted with at least one hydroxyl radical and at least one lower alkyl radical,
- or an alkyl or alkenyl chain of 4 to 8 carbon atoms substituted with at least one hydroxyl radical, at least one lower alkyl radical and at least one CF₃ radical.

8. Use of a compound according to any one of the preceding claims, for the manufacture of a medicinal product.

9. Use of a compound according to any one of Claims 1 to 7, for the manufacture of a medicinal product intended for the treatment:
- of dermatological complaints associated with a keratinization disorder which has a bearing on differentiation and on proliferation, such as simple acne, comedones, polymorphonuclear leukocytes, rosacea, nodulocystic acne, acne conglobata, senile acne and secondary acnes such as solar, medication-related or professional acne;
- of other types of keratinization disorder, such as ichthyosis, ichthyosiform states, Darier's disease, palmoplantar keratoderma, leukoplasias and leukoplasiform states, and cutaneous or mucous (buccal) lichen;
- of other dermatological complaints with an inflammatory and/or immunoallergic component, with or without cell proliferation disorder, and, in particular, all forms of psoriasis, whether this is cutaneous, mucous or ungual psoriasis, and even psoriatic rheumatism, or alternatively cutaneous atopy, such as eczema or respiratory atopy or alternatively gingival hypertrophy;
- of dermal or epidermal proliferations, whether benign or malignant and whether they are of viral origin or otherwise, such as common warts, flat warts and verruciform epidermodysplasia, oral or florid papillomatoses, T lymphoma and proliferations which can be induced by ultraviolet radiation, in particular in the case of basocellular and spinocellular epithelioma, as well as any pre-cancerous skin lesions such as keratoacanthomas;
- of other dermatological disorders such as immune dermatitis such as lupus erythematosus, immune bullosis and collagen diseases such as scleroderma;
- of dermatological or general complaints with an immunological component;
- of disorders of sebaceous function such as the hyperseborrhoea of acne or simple seborrhoea;
- of skin disorders due to exposure to UV radiation, ageing of the skin, whether it is light-induced or chronological ageing, or pigmentations and actinic keratoses, or any pathologies associated with chronological or actinic ageing;
- of cicatrization disorders or stretchmarks;
- of inflammatory complaints such as arthritis, of any complaint of viral origin on the skin or generally, such as Kaposi's syndrome;
- of ophthalmological disorders, in particular corneopathies;
- of cancerous or pre-cancerous states of cancers presenting or possibly being induced by vitamin D receptors, such as breast cancer, leukaemia, myelodysplasic syndromes and lymphomas, carcinomas of the Malpighian epithelial cells and gastrointestinal cancers, melanomas and osteosarcoma;
- of alopecia of various origins, in particular alopecia due to chemotherapy or radiation;
- of immune system complaints, such as autoimmune diseases, for instance type 1 diabetes mellitus, multiple sclerosis, lupus and lupus-type complaints, asthma, glomerulonephritis, selective dysfunctions of the immune system such as AIDS, or of immune rejection;
- of endocrine complaints;
- of complaints **characterized by** abnormal management of intracellular calcium, and of pathologies in which calcium metabolism is involved, such as muscular ischaemia;
- of vitamin D deficiencies and other mineral homeostasis complaints in plasma and bone, such as rickets, osteomalacia, osteoporosis, in particular in the case of menopausal women, renal osteodystrophy and parathyroid function disorders;
- of complaints of the cardiovascular system such as arteriosclerosis or hypertension, as well as non-insulin-dependent diabetes.

10. Pharmaceutical composition, **characterized in that** it comprises, in a pharmaceutically acceptable support, at least one of the compounds as defined in any one of Claims 1 to 7.

11. Composition according to Claim 10, **characterized in that** the concentration of compound(s) according to one of Claims 1 to 7 is between 0.0001% and 5% by weight relative to the total weight of the composition.

12. Cosmetic composition, **characterized in that** it comprises, in a cosmetically acceptable support, at least one of the compounds as defined in any one of Claims 1 to 7.

13. Composition according to Claim 12, **characterized in that** the concentration of compound(s) is between 0.001% and 3% by weight relative to the total weight of the composition.

14. Use of a cosmetic composition as defined in either of Claims 12 and 13, for body or hair hygiene.
